# EUROPEAN PATENT SPECIFICATION

(11) **EP 0 259 893 B1**
(45) Date of publication and mention of the grant of the patent: **22.01.1997**
(21) Application number: 87113386.4
(22) Date of filing: 14.09.1987
(51) Int. Cl.: A61K 45/06, A61K 38/19, A61K 39/00, A61K 51/00, G01N 33/48

(54) **Agents for removing advanced glycosylation endproducts**
Mittel zur Entziehung von Endprodukten der fortgeschrittenen Glycosylation
Agents pour éliminer les produits terminaux de la glycosylation avancée

(30) Priority: 12.09.1986 US 907747
(43) Date of publication of application: 16.03.1988
(73) Proprietor: THE ROCKEFELLER UNIVERSITY, New York, NY 10021 (US)
(72) Inventor: Vlassara, Helen, New York New York 10021 (US); Brownlee, Michael, New York New York 10021 (US); Cerami, Anthony, Shelter Island New York 11964 (US)
(74) Representative: Prato, Roberto

(56) References cited:
- WO-A-85/04169
- PROC. NATL. ACAD. SCI. USA, vol. 82, September 1985, pages 5588-5592; H. VLASSARA et al.: "High-affinity-receptor-mediated uptake and degradation of glucose-modified proteins: A potential mechanism for the removal of senescent macromolecules"
- JOURNAL OF CELLULAR BIOCHEMISTRY, VOL. 30, no. 2, 1986, New-York, US; A. CERAMI et al.: "Role of nonenzymatic glycosylation in atherogenesis"
- PROC. NATL. ACAD. SCI. USA, vol. 81, May 1984, pages 2684-2688; S. PONGOR et al.: "AGING OF PROTEINS: Isolation and identification of a fluorescent chromophore from the reaction of polypeptides with glucose"

## Description

### RELATED PUBLICATIONS

The Applicants are co-authors of the following articles directed to the subject matter of the present invention: "FUNCTION OF MACROPHAGE RECEPTOR FOR NONENZYMATICALLY GLYCOSYLATED PROTEINS IS MODULATED BY INSULIN LEVELS", Vlassara, Brownlee and Cerami, DIABETES (1986), Vol. 35 Supp. 1, Page 13a; "ACCUMULATION OF DIABETIC RAT PERIPHERAL NERVE MYELIN BY MACROPHAGES INCREASES WITH THE PRESENCE OF ADVANCED GLYCOSYLATION ENDPRODUCTS", Vlassara, H., Brownlee, M., and Cerami, A. J. EXP. MED. (1984), Vol. 160, pp. 197-207; "RECOGNITION AND UPTAKE OF HUMAN DIABETIC PERIPHERAL NERVE MYELIN BY MACROPHAGES", Vlassara, H., Brownlee, M., and Cerami, A. DIABETES (1985), Vol. 34, No. 6, pp. 553-557; "HIGH-AFFINITY-RECEPTOR-MEDIATED UPTAKE AND DEGRADATION OF GLUCOSE-MODIFIED PROTEINS: A POTENTIAL MECHANISM FOR THE REMOVAL OF SENESCENT MACROMOLECULES", Vlassara H., Brownlee, M., and Cerami, A., PROC. NATL. ACAD. SCI. U.S.A. (Sept. 1985), Vol. 82, pp. 5588-5592; "NOVEL MACROPHAGE RECEPTOR FOR GLUCOSE-MODIFIED PROTEINS IS DISTINCT FROM PREVIOUSLY DESCRIBED SCAVENGER RECEPTORS", Vlassara, H., Brownlee, M., and Cerami, A. JOUR. EXP. MED. (1986) (in press) "ROLE OF NONENZYMATIC GLYCOSYLATION IN ATHEROGENESIS", Cerami, A., Vlassara, H., and Brownlee, M., Journal of Cellular Biochemistry (1986), Vol. 30, pp. 111-120.

### BACKGROUND OF THE INVENTION

The reaction between glucose and proteins has been known for some time, and in its earliest manifestation, was identified in the appearance of brown pigments during the cooking of food. This phenomenon was identified by Maillard in 1912, who observed that glucose and other reducing sugars react with amino acids to form adducts that undergo a series of dehydrations and rearrangements to form stable brown pigments. Maillard L.C. (1912) C.R. Acad. Sci., Vol. 154, pp. 66-68.

This phenomenon was found in recent years to have its parallel in vivo. Accordingly, the nonenzymatic reaction between glucose and the free amino groups on proteins to form a stable amino 1-deoxy ketosyl adduct, known as the Amadori product, has been shown to occur with hemoglobin, wherein a rearrangement of the Amadori product formed at the amino terminal of the beta chain of hemoglobin by reaction with glucose forms the adduct known as hemoglobin A_{1c}. This initial reaction of the Maillard sequence was also found to occur with a variety of other body proteins, such as lens crystallins, collagen and nerve proteins. See Bunn, H.F., Haney, D.N., Gabbay, K.H. and Gallop, P.H., (1975) Biochem. Biophys. Res. Comm., Vol. 67, pp. 103-109; Koenig, R.J., Blobstein, S.H. and Cerami, A., (1977) J. Biol. Chem., Vol. 252, pp. 2992-2997; Monnier, V.M. and Cerami, A., (1983) MAILLARD REACTION IN FOOD AND NUTRITION, ed. Waller, G.A., American Chemical Society, Vol. 215, pp. 431-448; and Monnier, V.M. and Cerami, A., (1982) Clinics in Endocrinology and Metabolism, Vol. 11, pp. 431-452.

Moreover, brown pigments with spectral and fluorescent properties similar to those of late-stage Maillard products have also been observed in vivo in association with several long-lived proteins, such as lens proteins and collagen from aged individuals. An age-related linear increase in pigment was observed in human dura collagen between the ages of 20 and 90 years. See Monnier, V.M. and Cerami, A., (1981) SCIENCE, Vol. 211, pp. 491-493; Monnier, V.M. and Cerami, A., (1983) BIOCHEM. BIOPHYS. ACTA., Vol. 760, pp. 97-103; and Monnier, V.M., Kohn, R.R. and Cerami, A., "Accelerated Age-related Browning of Human Collagen in Diabetes Mellitus", (1984) PROC. NAT. ACAD. SCI., Vol. 81, pp. 583-587. Interestingly, the aging of collagen can be mimicked in vitro by the cross-linking induced by glucose; and the capture of other proteins in the formation of adducts by collagen, also noted, is theorized to occur by a cross-linking reaction, and is believed to account for the observed accumulation of albumin and antibodies in kidney basement membrane and cholesterol-bearing low density lipoprotein in the arterial wall. See, Brownlee, M., Pongor, S. and Cerami, A., (1983) J. EXP. MED., Vol. 158, pp. 1739-1744; and Kohn, R.R., Cerami, A. and Monnier, V.M., (1984) DIABETES, Vol. 33, No. 1, pp. 57-59. Cerami, A., Vlassara, H., and Brownlee, M., (1985) METABOLISM, Vol. 34, pp. 37-44.

In Pongor, S.M. et al, "Proc. Natl. Acad. Sci. USA, Vol. 81, pp. 2684-2688, May 1984", a fluorescent chromophore was isolated and identified which was found to be present in certain browned polypeptides such as bovine serum albumin and poly-L-lysine, and was assigned a structure 2-(2-furoyl)-4(5)-2-furanyl)-1H-imidazole (hereinafter "FFI"). The compound was found to exist in a tautomeric state and has incorporated in its structure two peptide-derived amine nitrogens. The incorporation of these amine nitrogens and two glucose residues in the compound suggested that its peptide-bound precursor may be implicated in the in vivo cross-linking of proteins by glucose which is observed in the late stage of the Maillard process. See, Chang, J.C.F., Ulrich, P.D., Bucala, R., and Cerami, A., (1985) J. Biol. Chem., Vol 260, pp. 7970-7974. This chromophore made possible the identification of the advanced glycosylation endproducts and assisted additional investigations seeking to clarify the protein aging process and if possible, to identify the specific chemistry involved to assist efforts to develop methods and agents for its inhibition. Developmental work by one of the inventors herein has resulted in the identification of certain compounds that demonstrate the ability to inhibit advanced glycosylation.

PCT International Application Published under the publication number WO85/04149, a Patent Application previously filed by the inventors herein, discloses the characterization and isolation of a fluorescent chromophore observed in proteins exposed to glucose over time, and whose fluorescent properties closely resemble those of a polypeptide after it undergoes advanced glycoslylation. The chromophore was structurally identified and named 2-furoyl-4 (5)-(2-furanyl)-1H-imidazole, and is believed to be one of the end products of extended non-enzymatic polypeptide glycoslyation in the state known a non-enzymatic browning (NEB). The Application discloses that measurement of this chromophore makes possible qualitative and quantitative assessment of the degree of aging.

Further work since the development of the inhibitors mentioned above has resulted in the identification of what appears to be an endogenous means for the in vivo elimination or removal of advanced glycosylation endproducts. This concept has been developed and is set forth herein, and it is accordingly to this purpose that the present Application is directed.

### SUMMARY OF THE INVENTION

In accordance with the present invention, agents are disclosed for the inhibition and treatment of protein aging in animals by stimulating the bodies of such animals to increase their recognition of and affinity for advanced glycosylation endproducts. In particular, phagocytic cells such as monocytes and macrophages are treated with an agent capable of causing the phagocytic cells to increase their activity of recognizing and removing macromolecules such as target proteins.

The present invention thus concerns a composition for promoting the sequestration and removal from the body of an animal of target macromolecules that have undergone advanced glycosylation comprising an agent capable of causing the body to increase its activity of recognizing and removing said macromolecules, characterized in that said agent is selected from the group consisting of an advanced glycosylation endproduct, an advanced glycosylation endproduct bound to a carrier, a monokine that stimulates the body to increase said recognizing and removing activity toward said target macromolecule, in combination with a co-stimulatory agent differing from the stimulatory agent.

The agents of the present invention comprise one or more stimulator compounds in turn, comprising a natural or synthetic advanced glycosylation endproduct alone or bound to a carrier, said carrier including a material selected from carbohydrates, proteins, synthetic polypeptides, lipids, bio-compatible natural and synthetic resins, antigens, and mixtures thereof. The stimulator compounds could include other advanced glycosylation endproducts that may be prepared from the reaction between sugars and other macromolecules, and monokines which stimulate phagocytic cells to increase their activity toward advanced glycosylation endproducts.

Accordingly, the stimulator compound may comprise the compound FFI bound to a protein such as albumin. Alternately, the stimulator compound may comprise a synthetically derived advanced glycosylation endproduct which is prepared, for example, by the reaction of glucose or glucose-6-phosphate with albumin. This reaction product can be used alone or with a carrier in the same fashion as the FFI-albumin complex.

A monokine that functions as a stimulator compound comprises the protein known as Tumor Necrosis Factor (TNF) and its variant discovered and isolated by one of the inventors herein and named "cachectin". This material may be administered alone or in conjunction with other stimulator compounds.

In addition, the stimulator compounds of the present invention may be administered in conjunction with materials identified hereinafter as "co-stimulatory agents". The coadministration of the stimulator compound with the co-stimulatory agents has been found to potentiate the activity of the former. Suitable co-stimulatory agents include monokines such as Interleukin-1 (IL-1) and gamma-interferon.

A treatment that, owing to the invention, may be practiced independently or conjointly with the above recited method, is the ex vivo treatment of the phagocytic cells to expose them to the stimulator compounds. For example, a patient may be given an extracorporeal blood treatment in which blood is diverted out of the body from the arterial and venous system and is directed through a device which contains stimulator compounds and/or co-stimulatory agents which are suitably positioned to come in contact with the phagocytic cells within the blood. The stimulator compounds and/or co-stimulatory agents may be immobilized or may be allowed to enter the flow of the body fluid.

In the instance where the treatment comprises the in vivo administration of the stimulator compound and/or stimulatory agents, such administration may be accomplished by known techniques, including oral techniques and parenteral techniques such as intradermal, subcutaneous, intravenous, or intraperitoneal injection, catheterization or other conventional means. The stimulator compounds or mixtures of them may be prepared in suitable pharmaceutical compositions for such administration.

In a further aspect of the present invention, phagocytic cells may be stimulated to increase their ability to recognize and remove target macromolecules by adjustment of the insulin level in the body fluid. In particular, artificial reduction of insulin levels may be achieved by dietary manipulation and/or by the use of pancreatic beta-cell suppression, conducted alone or in combination with the administration of the agents discussed above. Thus, this additional method exerts a positive effect on the activity of the phagocytic cells and promotes the increased uptake and elimination of advanced glycosylation endproducts in accordance with the present invention.

Several pathologies, such as age related or diabetes related hardening of the arteries, skin wrinkling, arterial blockage and diabetic retinal and renal damage are all the result of the excessive build-up or trapping that occurs as the presence of advanced glycosylation endproducts increases. Accordingly, a therapeutic method in which the invention may be used generally seeking to avert such pathologies, contemplates the administration of the agents of the present invention either directly or in suitable pharmaceutical compositions to stimulate the phagocytic cells to remove advanced glycosylation endproducts from the body with greater speed and efficiency, and to thereby avert the onset of the pathologies recited herein. Specific administrative protocols may vary and would be determined upon the specific instruction of qualified medical or veterinary practitioners.

The present method has particular therapeutic application as the Maillard process acutely affects several of the significant protein masses in the body, among them collagen, elastin, lens proteins, and the kidney glomerular basement membranes. These proteins deteriorate both with age (hence the application of the term "protein aging") and as a result of prolonged exposure to blood sugar and AGE formation. Consequently, the enhanced ability to remove glycosylation endproducts from the animal's system carries the promise of favorably treating the significant adverse effects of numerous pathologies including diabetes and of course, improving the quality and perhaps duration of animal life.

A further therapeutic application of the present invention lies in the area of immunology. In particular, advanced glycosylation endproducts generally, and FFI in particular, may be coupled to antigens, and phagocytic cells may then be exposed to this coupled complex to promote the uptake and digestion of this coupled complex by such cells. The phagocytic cells would then present the digested complex to the immune system of the animal of origin to elicit the development of antibodies to the particular antigen. In this manner, antigens that might not otherwise elicit an immunologically significant response could be made more immunologically reactive to develop effective defenses to the antigen. The foregoing method could be practiced in vivo or ex vivo, as, for example, the coupled complex including the advanced glycosylation endproduct or FFI could either be introduced into the body of the animal, or phagocytic cells could be isolated from the animal in extracorporeal fashion and thereby contacted with the coupled complex, after which the phagocytic cells with their increased receptors could be reintroduced into the animal's body to appropriately stimulate the immune system to develop antibodies.

A variant to the foregoing protocol contemplates the stimulation of the phagocytic cells to take direct action against the antigen. In this procedure, an antibody specific to the target antigen is labeled with or coupled to an advanced glycosylation endproduct such as FFI, and the labeled/coupled material is then introduced in vivo to promote the arousal of the activity of the phagocytes toward this material. The labeled/coupled material would bind to the target antigen and the resulting complex would be attacked by the phagocytes that recognize the AGE or FFI. Attack would occur either directly or by the secretion of a monokine such as cachectin to cause the necrosis of the antigen. The phagocytes could be preliminarily activated by in vivo or ex vivo means as described earlier. This protocol, like the one described above, possesses particular utility as a possible treatment for Acquired Immune Deficiency Syndrome (AIDS) and other tumorous or viral infections.

In addition, the present invention contemplates certain diagnostic applications, including the development of an assay system for screening of potential drugs effective to act as agents to stimulate the activity of particular phagocytic cells against advanced glycosylation endproducts. In one instance, a prospective test drug could be administered to a macrophage sample to determine its stimulatory effect, with control samples receiving, respectively, a known stimulator compound such as those listed above, and no stimulation whatsoever. Further, a particular phagocytic cell sample could be investigated to determine the agents from among those known that are most effective in stimulating such cellular activity, if such stimulation is possible, by the inoculation of a series of identical sample colonies with various of the known agents recited above, with such agents being appropriately labeled by a radioactive indicator or otherwise, to chart the activity or progress in stimulation, by the uptake of such agents by the particular cellular colony. In such manner, the colony exhibiting the greatest uptake and disposal of labeled advanced glycosylation endproducts would identify the corresponding agent that is most effective in this stimulatory capacity.

By the above diagnostic techniques, cellular colonies capable of stimulation could be determined, and in the instance where such capability is in evidence, the colonies could be further examined to determine whether any discrimination in the particular agent capable of achieving such stimulation is in evidence.

Additional diagnostic applications relate to the study of a variety of parameters attending the recognition and removal of advanced glycosylation endproducts by phagocytic cells and the significance of this phenomenon in relation to the functioning of the animal's system. In a first embodiment, phagocytic cells such as macrophages may be removed from the animal's body and activated ex vivo by exposure to advanced glycosylation endproducts. These activated phagocytic cells may then be radiolabeled as with Technicium and thereafter reintroduced to the animal and allowed to circulate through the animal's system, while being radioimaged to note the final location of the cells. In this manner, the location of concentrations of advanced glycosylation endproducts in the animal's body could be identified. This technique is particularly useful in identifying undesirable concentrations of advanced glycosylation endproducts, such as atheromatous plaques. In such manner, the location of the systemic malfunction could be identified.

Also, the condition of the system for the removal of advanced glycosylation endproducts from the body could be measured by the preparation of radiolabeled advanced glycosylation endproducts and the administration of these radiolabeled materials to the body to determine the time required for their recognition, uptake and elimination. Such measurement could then be compared against standard measurements determined by testing normal systems under the same parameters. The foregoing test could, for example, be performed as a test for diabetes, or other disorders that would adversely effect the operability of the AGE removal system of the body. Alternately, this method may likewise be practiced in extracorporeal fashion by removing phagocytic cells from the body and testing them for their efficiency and rate of operation ex-vivo with radiolabeled advanced glycosylation endproducts.

A further diagnostic technique could measure the presence of pathology as a function of the state of activation of the phagocytic cells in the body of the animal under investigation. Thus, macrophage cells could be exposed to particular radiolabeled advanced glycosylation endproducts known to be found in connection with certain pathologies, and the state of stimulation of the phagocytic cells could then be observed, by comparison against suitably developed norms, to determine whether the phagocytic cells are in a state of stimulation, and if so, as to the probable source of such stimulation. Thus, the presence of particular AGEs in the body reflect correspondingly particular pathologies, and the observation of the phagocytic cells and the determination of their sensitivity and increased stimulation with respect to particular advanced glycosylation endproducts would suggest the existence of a particular pathology.

Accordingly, it is a principal object of the present invention to provide a method to allow improved sequestration and removal of advanced glycosylation endproducts from animal systems.

It is a further object of the present invention to provide a method as aforesaid which is characterized by the stimulation of phagocytic cells to increase their affinity and capability for the binding, uptake and degradation of advanced glycosylation endproducts.

It is a yet further object of the present invention to provide agents capable of stimulating phagocytic cells to bind, take up and degrade advanced endproducts in the method as aforesaid.

Other objects and advantages will become apparent to those skilled in the art from a consideration of the ensuing description which proceeds with reference to the following illustrative drawings.

### BRIEF DESCRIPTION OF THE DRAWINGS

FIGURE 1 is a graph depicting the relative binding and uptake of red blood cells modified with various agents, including one of the agents of the present invention.

FIGURE 2 is a graph illustrating the competitive inhibition in red blood cell binding caused by the introduction into a sample of an agent in accordance with the present invention.

FIGURE 3 illustrates the binding and uptake by monocytes of red blood cells that have been modified by reaction with a variety of sugars.

FIGURE 4 is a line graph illustrating the half-life of red blood cells that have been labeled and modified by association with an agent in accordance with the present invention, as compared with a control.

FIGURE 5 is a bar graph illustrating the comparative uptake and degradation of advanced glycosylation endproducts by mouse macrophages exposed to various stimulator compounds.

FIGURE 6 is a bar graph illustrating data similar to that set forth in FIGURE 5, with respect to one day old human monocytes.

FIGURE 7 is a bar graph illustrating similar comparative experiments conducted with human monocytes wherein the co-stimulatory agent gamma interferon was also added and tested.

FIGURE 8 is a graph illustrating the effect of insulin on the binding capabilities of mouse macrophage cells, wherein a normal sample and an alloxan induced diabetic macrophage sample were compared.

FIGURE 9 is a graph similar to FIGURE 8, illustrating a comparison between a normal or control sample of human macrophages with those of hypo- and hyper- insulinaemic diabetic macrophages as to the binding capability of each of the samples.

FIGURE 10 is a graph similar to FIGURE 9, making a comparison between the same macrophage samples as to their ability to degrade advanced glycosylation endproducts.

### DETAILED DESCRIPTION OF THE PREFERRED EMBODIMENT

In accordance with the present invention, a composition and associated methods have been developed for enhancing the removal of advanced glycosylation endproducts in animals, to treat protein aging and thereby inhibit the adverse effects thereof. Specifically, phagocytic cells such as monocytes and macrophage cells are exposed to one or more agents or stimulator compounds which enhance the ability of such phagocytic cells to recognize, bind and degrade advanced glycosylation endproducts.

The present invention is predicated upon the observation that monocytes and macrophage cells have the ability to recognize, remove and degrade macromolecules that have undergone glucose-mediated damage and thus have undergone advanced glycosylation endproduct formation. In particular, it has been determined that monocytes and macrophages have a specific surface receptor for the glucose-altered macromolecules that allows the cells to perform their recognition, removal and degrading functions with respect thereto.

Previous work with respect to glucose-mediated changes in proteins has determined that glucose reacts with such macromolecules and leads to changes in their properties which interfere with their normal function. Glucose is known to react specifically with the amino groups of proteins without the aid of enzymes to cause them to become crosslinked or to covalently bind to and thereby trap other proteins.

In particular, glucose may initially react with an amino group on a protein and thereby form what is known as a reversible Schiff base adduct. The adduct then rearranges to form a more stable, but still reversible Amadori product. The Amadori product, which is an initially glycosylated protein may then undergo further reactions, which in one instance, include the reaction with a second glycosylated amino group of a protein, whereby a crosslink is formed. The result of this reaction is the conversion of the two glucose moieties and their amino groups into a compound identified previously by one of the inventors herein and referred to earlier as FFI. This compound is part of a complex family of advanced glycosylation endproducts that exhibit yellow and fluorescent appearance. The presence of this compound has been confirmed by the in vitro reaction of glucose and proteins, whereby the resulting mass is observed to become yellow to brown in color. These advanced glycosylation endproducts form simply from the reaction of sugars with proteins and other macromolecules, including DNA.

The Amadori product can also react with a second glucose, and the resulting doubly glycosylated derivative can then react directly with the amino group of a non-glycosylated protein to form an advanced glycosylation endproduct (AGE) and to thereby link the proteins together. This phenomenon has been termed "trapping" and has been demonstrated in vitro by the reaction of collagen, a normally insoluble, structural protein, and low-density lipoprotein, which is a circulating, soluble protein, as well as collagen with albumin and IgG. See Brownlee, M., et al., DIABETES, Vol. 34, pp. 938-941 (1985); Brownlee, M., Pongor, S., and Cerami A., J. EXP. MED., Vol. 158, pp. 1739-1744 (1983).

Glucose-mediated crosslinking and trapping of proteins is a normal process in the body which over time leads to pathology in many tissues and organs. Internal crosslinking of proteins or crosslinking of two adjacent proteins may change the mechanical properties of structural proteins. Changes in the immunologic, enzymatic, physical and other properties as a result of crosslinking and trapping are also known. For example, it has been observed that the abnormally high levels of glucose in the blood of diabetics leads to an abnormal increase in the formation of crosslinks and trapped proteins, and it is postulated that this may be responsible for the increased morbidity and mortality of the disease.

Trapping leads to an abnormal build up of proteins in abnormal locations which can lead to pathology. Examples of the pathology that can be caused by glucose-mediated crosslinking and trapping includes the attachment of lipoprotein and other plasma proteins to the walls of coronary arteries, and its consequent build up of proteins and cholesterol to cause arterial blockage and heart attacks. Similarly, crosslinking of collagen in the arterial wall can change the mechanical properties of the arterial wall by stiffening its structure and thereby causing circulatory problems. Thickening of basement membranes of smaller blood vessels in the body and in the kidney resulting from trapping and crosslinking leads to peripheral vascular disease and thickening of the kidney basement membrane with subsequent loss of kidney function (nephropathy). In addition, thickening of vessel walls in the brain leads to reduced blood flow and can contribute to the onset of senility.

Crosslinking of collagen and other macromolecules in the skin may lead to wrinkling and other changes, as well as changes in the diabetic eye and particularly damage to the lens and to the vessels of the retina, which latter event leads to a loss of visual acuity and ultimately, to blindness. Finally, glucose is known to react with DNA, and experiments have shown that AGE-DNA can be mutagenic in bacteria.

As noted earlier, phagocytic cells are capable of recognizing and removing abnormal macromolecules by means of receptors on their surfaces which recognize specific chemical structures and bind to them. Once the abnormal macromolecule is recognized in this way, the phagocytic cell may internalize the macromolecule or particle containing the abnormal macromolecule and may then degrade it. In some instances, the phagocytic cell may in addition secrete enzymes and other factors to help degrade the molecule or particle extracellularly if it cannot be internalized. After the damaged protein is removed, new growth of normal tissue can ensue, and normal function of the affected area may resume.

Phagocytic cells in the body comprise numerous types of white blood cells. One type of white blood cell, the monocyte, is produced in the bone marrow, and circulates briefly in the blood and thereafter enters the tissues where it becomes a macrophage. Exposure of the phagocytic cell either as a monocyte or a macrophage, to certain molecules can regulate the appearance on the surface of the cell of receptors for these molecules.

Thus, the present invention is predicated on the discovery that the phagocytic cells including monocytes and macrophages can be modified by exposure to certain agents or stimulator compounds that potentiate the capability of these cells with respect to their recognition and affinity for, and capability to degrade, advanced glycosylation endproducts. In particular, the exposure of these cells to certain stimulator compounds has been found to increase the number of receptors developed on these cells and to thereby increase the capacity and efficiency of these cells with respect to the recognition and degradation of advanced glycosylation endproducts.

Accordingly the method of the present invention generally comprises exposing the animal body to certain agents or stimulator compounds, which cause the body, and its phagocytic cells, in particular, to become activated and to increase its recognition and removal of target macromolecules that have undergone advanced glycosylation.

Suitable stimulator compounds useful in the present invention comprise materials including advanced glycosylation endproducts, either naturally or synthetically formed, which may be employed alone or bound to a carrier.

Suitable stimulator compounds include the compound FFI bound to a carrier protein such as the protein albumin. The stimulator compound may also comprise a synthetically derived advanced glycosylation endproduct which is prepared, for example, by the reaction of a protein or other macromolecule with a sugar such as glucose, glucose-6-phosphate, or others. This reaction product could be used alone or could be combined with a carrier in the same fashion as the FFI-albumin complex.

The carrier may be selected from the group consisting of carbohydrates, proteins, synthetic polypeptides, lipids, bio-compatible natural and synthetic resins, antigens and mixtures thereof.

As used herein, the term "antigen" includes various invasive stimuli that may comprise or cause the onset of pathology or other organic disability, such as protein and lipid fragments, bacteria, viruses and/or other organisms of similar origin and effect.

Stimulator compounds also include monokines which stimulate phagocytic cells to increase their activities toward advanced glycosylation endproducts.

A particular monokine that functions as a stimulator compound comprises the protein known as Tumor Necrosis Factor (TNF) and its variant discovered and isolated by one of the inventors herein and named "cachectin". This material may be administered alone or in conjunction with other stimulator compounds.

In addition, the stimulator compounds of the present invention may be administered in conjunction with materials identified hereinafter as "co-stimulatory agents". The coadministration of the stimulator compound with the co-stimulatory agents has been found to potentiate the activity of the former. Suitable co-stimulatory agents include monokines such as Interleukin-1 (IL-1) and gamma-interferon.

With regard to the preparation of the stimulator compound comprising FFI coupled to a carrier molecule such as albumin, the synthetic compound FFI-hexanoic acid may be used in its preparation. Thus, a water-soluble carbodiimide is used to attach the acid moiety of the FFI-hexanoic acid to an amino group on the protein. This conjugate, after purification, is used in vitro to stimulate macrophages. After incubation for 4 to 24 hours, it can be shown that such macrophages will more actively bind, internalize, and degrade AGE-albumin.

The adverse effects of the buildup of advanced glycosylation endproducts in animals result in such conditions as age- or diabetes- related hardening of the arteries, skin wrinkling, arterial blockage and diabetic retinal and renal damage from the excessive buildup or trapping that occurs as advanced glycosylation endproducts increase in quantity. Accordingly, a therapeutic method seeking to avert pathologies caused at least in part by the accumulation of advanced glycosylation endproducts in the body comprises the administration of the agents of the present invention either directly or in suitable pharmaceutical compositions to stimulate the body to increase its activity toward the recognition and removal of such advanced glycosylation endproducts. In particular, the agents are administered to stimulate the phagocytic cells in the body to increase their activity toward the recognition and removal of advanced glycosylation endproducts so that such removal occurs with greater speed and efficiency. Specific administrative protocols would vary and would be determined upon the instruction of qualified medical or veterinary practitioners.

Accordingly, the present invention also includes suitable pharmaceutical compositions for use in the therapeutic methods of the invention, comprising the agents of the present invention prepared in a suitable pharmaceutically acceptable carrier. Such carriers are known and may vary in composition and/or concentration depending upon the manner of administration, i.e. oral, parenteral, etc.

The present invention will be better understood from a consideration of the following illustrative examples and data, that confirm the activities of the phagocytic cells and their relationship to the stimulator compounds discovered in accordance herewith.

### EXAMPLE I

In the investigation which follows, the existence in vivo of the clearance system of advanced glycosylation endproducts was confirmed and studied, and the principles of the present invention were established.

### MATERIALS AND METHODS

RBC preparation: Human blood (2.0 ml) from normal, healthy adult volunteers was collected in heparinized tubes. Following removal of plasma and buffy coat, the RBC's were washed four times with 10 vol of 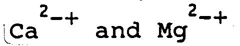 free phosphate-buffered saline (PBS), pH 7.4, and were resuspended in Dulbecco's modified Eagle medium.

Opsonized RBC preparation: 0.1 ml of a 15% RBC suspension from a B⁺ donor was added to 0.5 ml of a high-titer anti-D serum and was incubated at 37°C for 30 minutes. Following incubation, cells were washed three times with PBS (GIBCO) and were resuspended in 3 ml of Dulbecco's modified Eagle medium.

FFI-RBC preparation: The specific advanced glycosylation endproduct (AGE) [2-(2-furoyl)-4(5)-(2-furanyl)-1H_-imidazole]-hexanoic acid (FFI-HA) was prepared as described previously (Pongor et al., PNAS (1984) Vol. 81, pp. 2684-2688). In brief, furylglyoxal hydrate (10 mmol) in 3:1 dioxane/water was treated with 6-amino hexanoic acid (15 mmol) and triethylamine (15 mmol) and stirred at 25°C for 1 hour. After the addition of concentrated aqueous ammonia, the mixture was diluted with 5% NaH₂PO₄ and extracted with CH₂Cl₂, washed with brine and filtered through activated carbon and MgSO₄. The crude product was purified by medium pressure chromatography on silica gel yielding FFI-HA as straw-colored flakes (m.p. 105-106°C). Freshly washed normal RBC's were resuspended in PBS, with and without 10 mM water soluble 1-cyclohexyl-3-[2-(4-morpholinyl)-ethyl]-carbodiimide (CMC) (Vlassara, et al., (1985), Vol. 82, pp. 5588-5592), to which FFI-HA was added at different concentrations (10-100 M). The mixtures were incubated under continuous mixing for 1 hour at room temperature. Parallel mixtures containing RBC's alone in PBS or RBC's and carbodiimide (10 mM) were treated identically as controls. After 3 washes the cells were resuspended in 1 mM glycine in PBS and left to incubate for 30 minutes. Following three washings with PBS the cells were suspended in RPMI prior to phagocytosis assay, at an 100 excess ratio to monocytes.

Glycosylated-RBC preparation: In order to produce nonenzymatically glycosylated erythrocytes, glucose, glucose-6-phosphate, xylose and arabinose were added to freshly isolated normal human red cells suspended in Dulbecco's modified Eagle medium at 100 mM concentrations and incubated for 48 hours at room temperature. RBC suspensions without sugars added were used as control cells. Following the incubation, the cells were washed three times with PBS and were suspended in RPMI.

AGE-BSA was prepared by incubating bovine serum albumin (BSA) in 50 mM glucose at 37°C for 6 weeks, in the presence of protease inhibitors (PMSF 1.5 mM, EDTA 0.5 mM) and antibiotics (penicillin 100 U/ml, gentamicin 40 mg/ml) See, Vlassara et al., supra.)

Human monocyte preparation: The buffy coat from 100 ml fresh human blood was diluted two-fold with saline, 1 mM EDTA, pH 7.4, and mononuclear cells were separated from other elements of the blood by centrifugation on Ficoll-Hipoque-Paque gradients as described before (Gimelig-Meyling et al. (1950), J. IMMUN. METHODS, Vol. 33, p. 1). The mononuclear cells were washed three times in cold RPMI 1640 (GIBCO) to remove platelets, and the cells were suspended in RPMI made 10% in normal human serum. To obtain suspensions of monocytes for use in these experiments, the Percoll purification method described previously was used (Gimelig-Meyling supra.). Percoll was brought to isotonicity by the addition of 0.1 vol. of 10-X concentrated PBS. One ml of normal human serum, 14.7 ml PBS, and 22 ml isotonic Percoll was mixed in sterile 50-ml centrifuge tubes and centrifuged for 25 minutes at 18,000 rpm at 5°C in a Sorvall SS-34 rotor. Five ml of the mononuclear suspension were layered on the resulting gradients and the tubes were centrifuged at 1,500 g for 25 minutes at 5°C in a swinging bucket rotor. The resulting bands were detected by light scattering and band I, corresponding to monocytes was transferred and cultured in screw cap Teflon jars (Savillex, Minnetonka, MN), at 10⁶/ml in RPMI with 12.5% human serum at 37°C in 5% CO₂ for 5-7 days. Cell viability was assessed by trypan blue exclusion (GIBCO) and plating efficiency of phagocytes on a tissue-culture plastic surface was measured by a hemacytometer before and after attachment. When ready for use, aliquots of 10⁶ monocytes were plated in sterile petri dishes containing precleaned round coverslips and incubated for 2 hours at 37°C in 5% CO₂.

Phagocytosis assay: Before the addition of RBC's the monocyte cultures were washed twice with RPMI 1640. The various red cell suspensions were added to each well at a 100-fold excess to the monocytes and were allowed to incubate at 37°C and 5% CO₂ for up to 2 hours. At that point the coverslips were removed from the wells, washed three times with RPMI to remove nonadherent material, placed into clean wells and fixed with 1.25% glutaraldehyde in PBS for 30 minutes. To differentiate surface-attached from ingested erythrocytes, a hypotonic solution (PBS diluted 1:4 in H₂0) was added to selected wells for 10 seconds, followed by the addition of fixative. To read the assay, duplicate coverslips were counted using 40X phase microscopy. At least 300 monocytes from three or more randomly selected fields were counted per well. The data were expressed as the number of positive monocytes (monocytes with erythrocytes attached or ingested) per 100 monocytes (percent binding). Ingestion index was also determined as the number of ingested or adhered red cells per positive monocyte (Bianco et al. (1976) J. EXP. MED., Vol. 144, p. 1531).

FFI-RBC 1/2 life assay: BALB/c inbred mice were bled by cardiac puncture yielding approximately 2.0 ml of blood. Red cells were washed with large volumes of divalent cation-free PBS and coupled with FFI-HA as described above in the presence of 10 mM CMC. In addition, RBC's incubated in CMC alone or PBS alone were used as controls. Subsequently all cell suspensions were labeled with ⁵¹Cr by adding 0.2 mCi Na₂[⁵¹Cr]O₄ to 2 ml of 50% packed RBC in RPM1-1640 medium for 1 hour at 37°C. The labeled cells were washed at least four times to remove unbound isotope. Twelve BALB/c mice were then injected intravenously with 200 l RBC suspension. Each sample was administered in three BALB/c mice. At appropriate time intervals the mice were bled (0.2 ml) and radioactivity levels were measured by counting.

### RESULTS

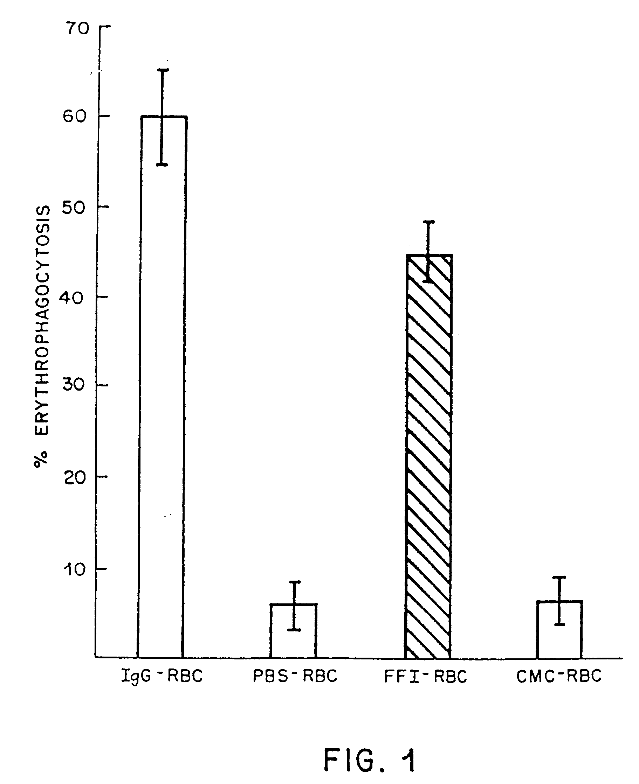

Maximum binding of red cells was observed on Day-7 of monocyte incubation in vitro. Maximum binding and endocytosis of FFI-RBC was complete within 30-45 minutes while opsonized cells were maximally bound within 15 minutes. At the end of one hour incubation of FFI-coupled RBC's with cultured human monocytes, per cent phagocytosis and phagocytic index were estimated. As shown in Figure 1, % erythrophagocytosis of FFI-modified red cells (55%) and IgG-coated red cells (70%) were significantly higher than that of control PBS-treated cells (4%). Similarly the phagocytic index of FFI-treated RBC's was greatly elevated (3.4) as compared to normal controls (1.2).

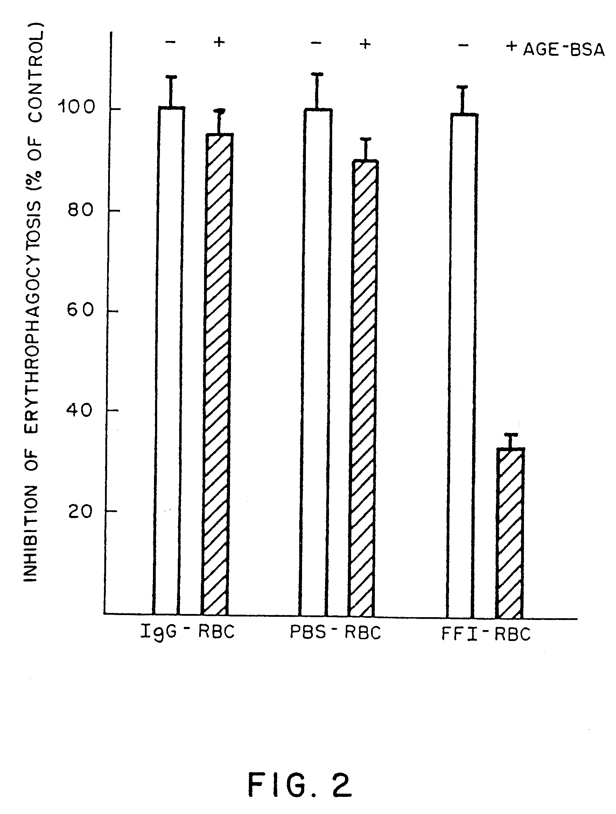

In order to establish the specificity of the interaction of FFI-RBC's with the human monocytes, competition experiments were carried out in which binding and ingestion of red cells was observed in the absence and presence of AGE-BSA, prepared as described in Methods (Vlassara et al., supra.). As shown in Figure 2, the addition of AGE-BSA at concentrations of 500 g/ml inhibited the FFI-RBC binding by more than 70% of the control. In contrast, AGE-BSA did not inhibit binding and phagocytosis of opsonized or PBS-treated red cells, even at maximal concentrations (1 mg/ml). These data suggested that FFI-modified red cells were recognized and bound specifically by the monocyte AGE-binding site.

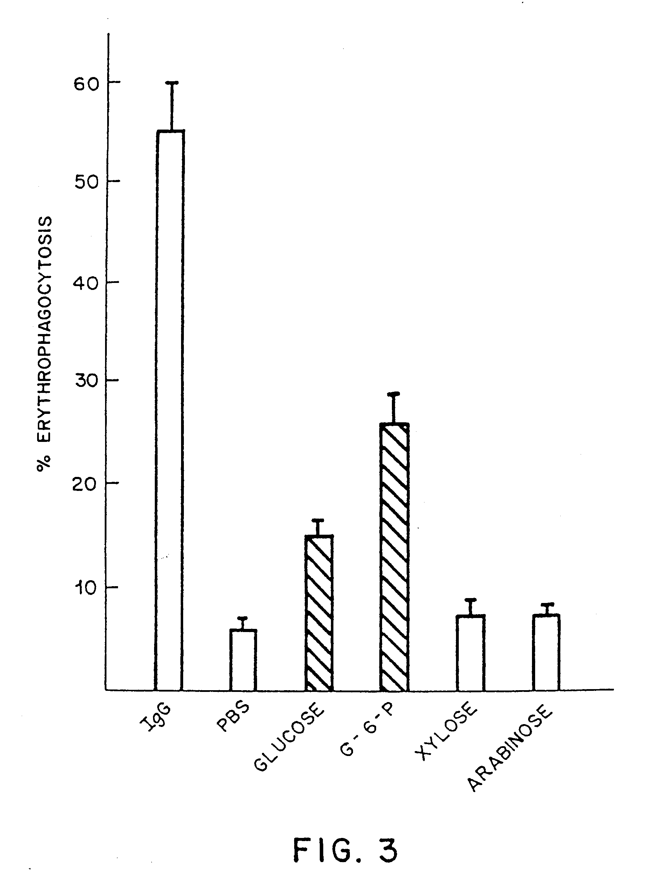

Advanced glycosylation endproduct (AGE) formation was then induced on red cell surfaces by 48 hour incubation in Dulbecco's modified Eagle medium containing different sugars, such as glucose, glucose-6-phosphate, xylose and arabinose, at 100 mM concentrations, at room temperature. At the end of this period, the media demonstrated no evidence of cell lysis, and red cells themselves appeared microscopically indistinguishable from the controls. As demonstrated by RIA (the method of Chang et al., (1985) J. BIOL. CHEM., Vol. 260, pp. 7970-7974), all cells incubated in sugar underwent formation of a significant amount of advanced glycosylation endproducts on their cell membrane, as compared to the controls. At this point RBC's from all groups were subjected to normal human monocyte phagocytosis assay. As indicated in Figure 3, glucose-incubated RBC's showed a 15% binding and ingestion and G6P-treated RBC's at 26%, as compared to 6% binding and ingestion of control PBS-RBC's. Much lower level of binding were noted with red cells preincubated with xylose and arabinose (7.5% each).

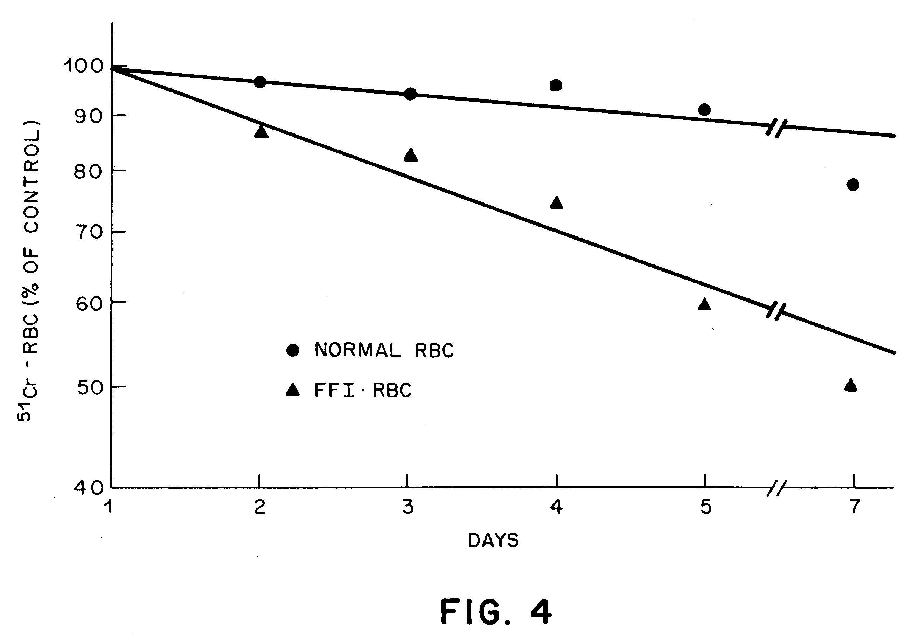

In order to determine whether autologous red blood cells modified by an AGE such as FFI-HA can be recognized by macrophages in vivo and be removed from the circulation faster than the non-modified cells, presumably by the same AGE-receptor present on hepatic and splenic macrophages, BALB/c inbred mouse red cells were treated either with FFI-HA, as described in Methods, or PBS alone for one hour at room temperature. As an additional control, mouse cells were treated with carbodiimide alone (10 mM). Following ⁵¹chromium-labeling, all three groups of cells were reinjected intravenously into syngeneic mice and erythrocyte radioactivity was monitored for 20 days. An approximate 9% of initial radioactivity recovered from all groups within the l hour of the injection was recovered in the serum fraction, presumably due to traumatic hemolysis caused by the ex vivo handling, since it was reduced to 0.4% within the first 24 hours. As shown in Figure 4, the half-life of FFI-treated red cells was reduced to 7 days, as compared to the control untreated cell half-life of approximately 20 days. Similarly, cells treated with carbodiimide alone had nearly normal in vivo time course.

### DISCUSSION

The above tests extend previous observations on the recognition of advanced glycosylation endproducts (AGE) by a specific monocyte/macrophage receptor, and present evidence that such adducts, once attached chemically or formed in vitro on the surface of intact human cells, can induce cell binding and ingestion by normal human monocytes. It is demonstrated that the presence of AGE on the cell surface in significant amounts over the unmodified cells leads to their shortened survival in vivo, presumably due to the more rapid removal of these AGE-cells by the splenic and hepatic phagocytic cells. The mechanism of recognition and removal of such modified cells appears to be mediated via the specific macrophage AGE-receptor, as shown by the competitive inhibition only of AGE-red cell binding in the presence of large excess of AGE-BSA (Vlassara et al., supra.).

With age, nonenzymatically glycosylated proteins normally undergo further modifications and rearrangements leading to the formation of increasing amounts of AGE's, as was shown in normal human dura collagen (Monnier et al. (1984) PNAS, Vol. 81, pp. 583-587). One specific endproduct, FFI, has been identified in vivo on normal human serum albumin and globin (Pongor et al., (1984) PNAS, Vol. 81, p. 2684). The AGE-receptor recognition increases with the amount of AGE, and can therefore preferentially recognize a time-dependent signal for the removal of senescent macro-molecules (Vlassara et al. supra.).

Nonenzymatic glycosylation of erythrocyte membrane proteins has been shown previously (Miller et al., (1980) J. CLIN INVEST. Vol. 65, pp. 896-901) to occur primarily at the lysine residues of all the major protein bands without distinction and is enhanced in diabetes, while it is decreased in hemolytic anemia. These findings have suggested that the modification of membrane proteins, other than by blood glucose, depends not only on glucose concentration, but also on erythrocyte age. The foregoing experiments demonstrate that AGE is present on normal intact red blood cells, which may be responsible in part for daily removal of erythrocytes from the circulation by the monocyte/macrophage AGE-receptor. Further, AGE accumulation can be accelerated by exposure to high glucose levels, which in turn leads to increased monocyte AGE-receptor binding and ingestion of glucose-modified red cells, and this may play a role in the moderately shortened erythrocyte survival in diabetics (Peterson, C.M., et al., (1977) ANN. INT. MED., Vol. 86, pp. 425-429).

The difference in erythrocyte binding between glucose- and glucose-6-phosphate - treated cells is probably due to the higher amount of AGE formed, owing to the presence of a higher percentage of more reactive open ring structures in the incubation mixture in the case of glucose-6-phosphate. Surprisingly, even though xylose and L-arabinose both can react faster with protein amino groups than glucose, as shown by the amount of AGE formed, they did not induce binding and uptake above the normal control cells. This phenomenon warrants further investigation, however it is probably due to formation of glycosylation products which are not recognized by the AGE or any other monocyte receptor.

### EXAMPLE II

The following series of experiments were performed to measure the ability of agents to stimulate phagocytic cells to stimulate uptake and degradation of endproducts (AGEs).

Accordingly, a number of AGEs were prepared using the same procedure as disclosed in Example I, above. Thus, FFI-HA was prepared as described and quantities were bound to both human and bovine albumin. A water soluble carbodiimide was used to attach the acid moiety of the FFI-HA to an amino group on the protein. After preparation, the conjugate was purified and then used in vitro to stimulate macrophages, by incubation for from 4 to 24 hours. The AGEs that were to be observed for uptake and degradation were appropriately radiolabeled so that they could be traced. Thereafter, the stimulated macrophages were tested by exposure to the radiolabeled AGEs following exposure to various agents to measure the effect that these agents had on the ability of the macrophages to take up and degrade the labeled AGEs. The above procedures and the studies that follow conform to the protocol employed by Vlassara et al., supra.

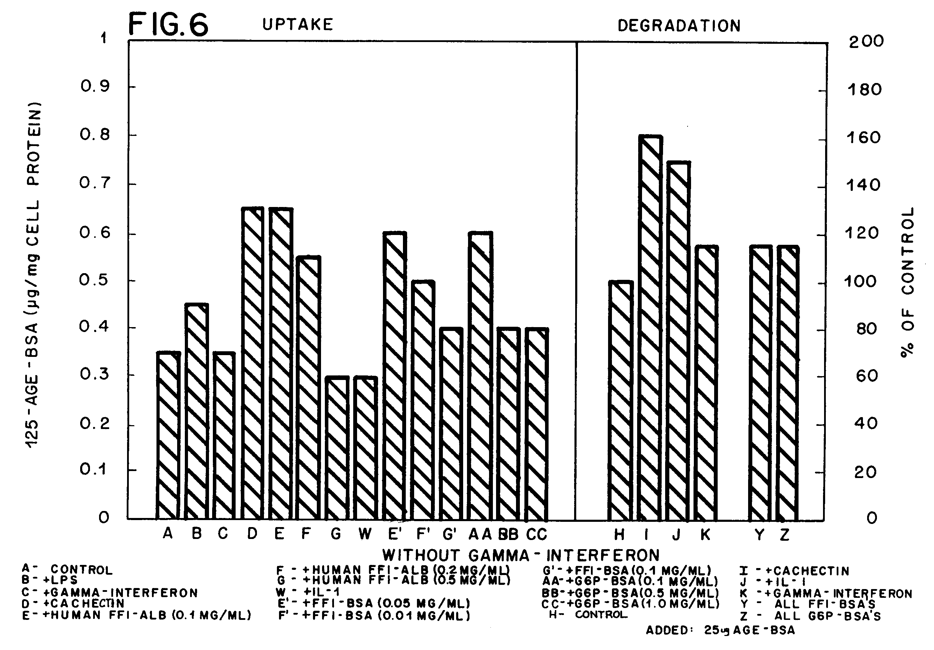

Thus, Figure 6, Part I, shows the uptake of AGE-BSA by human monocytes after stimulation with certain agents in the absence of gamma-interferon. The FFI-human albumin at 0.1 and 0.2 mg/ml had a stimulatory effect on the uptake system (bars E and F, respectively) as compared to the control (bar A). FFI-bovine albumin is also stimulatory (bars E', F', and G'). Gamma-interferon alone (bar C), or lipopolysaccharide alone (bar B) or Interleukin-1 alone (bar W) have no stimulatory effect.

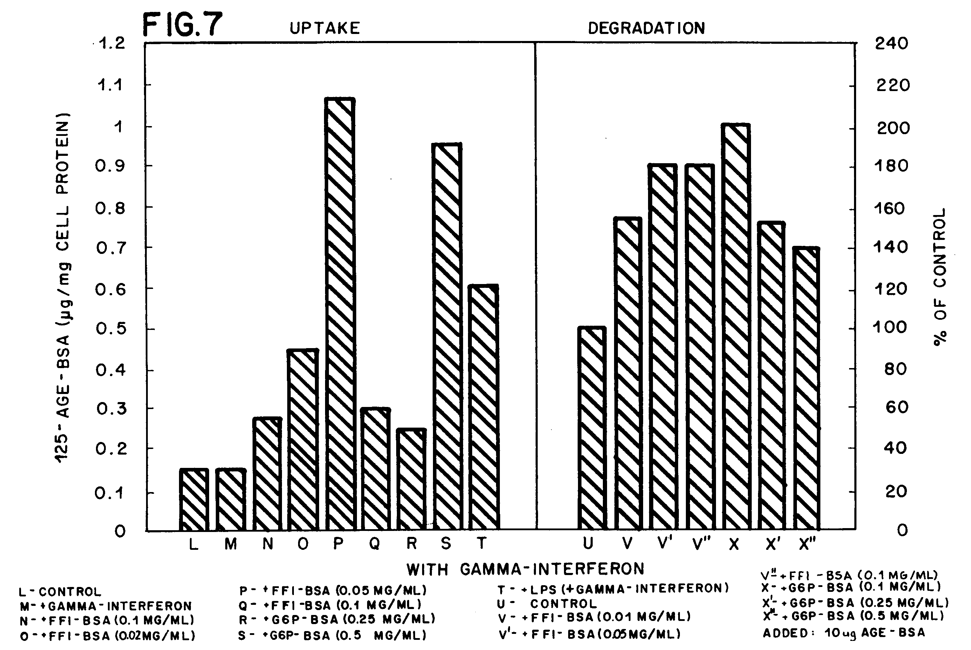

Figure 6, Part II, shows the degradation of AGE-BSA by human monocytes in the presence of these agents, in the absence of gamma-interferon. The FFI-BSA preparations are slightly stimulatory (bar Y). In Figure 7, Part 1, the effect of stimulation on the uptake of AGE-BSA by the same agents in the presence of 10 micrograms/ml of human gamma-interferon is shown. As can be seen, gamma-interferon greatly potentiates (up to 8-fold) the stimulation by 0.01, 0.02, and 0.05 mg/ml of FFI-BSA (bars N, O, and P). Degradation is also enhanced in the presence of these agents plus gamma-interferon (Figure 7, bar V).

It has also been demonstrated that monocyte or macrophage cells can also be stimulated by AGE-carrier molecules which result in cells with enhanced ability to bind, internalize and degrade other AGE-molecules. AGE-carrier molecules are made, for example, from the reaction of glucose or glucose-6-phosphate with albumin. After purification of the reaction product, the AGE-albumin uptake of AGE-macromolecules demonstrated as in (A) above. AGE-BSA (prepared from the incubation of glucose-6-phosphate with albumin for 6-8 weeks) at 0.1 mg/ml has a stimulatory effect on AGE-BSA uptake by human monocytes (Figure 6, bar AA), and shows a slight stimulation at higher concentrations (bars BB and CC). Figure 7, bar S, shows that in the presence of gamma-interferon, 0.5 mg/ml of AGE-BSA made from glucose-6-phosphate, greatly stimulates uptake of AGE-BSA by macrophages. The lower concentrations show a slight stimulatory effect (bars Q and R).

Degradation of AGE-BSA by human monocytes is also stimulated by AGE-BSA in the presence of gamma-interferon (Figure 7, bar X). Without interferon, stimulation is slight (Figure 6, bar Z).

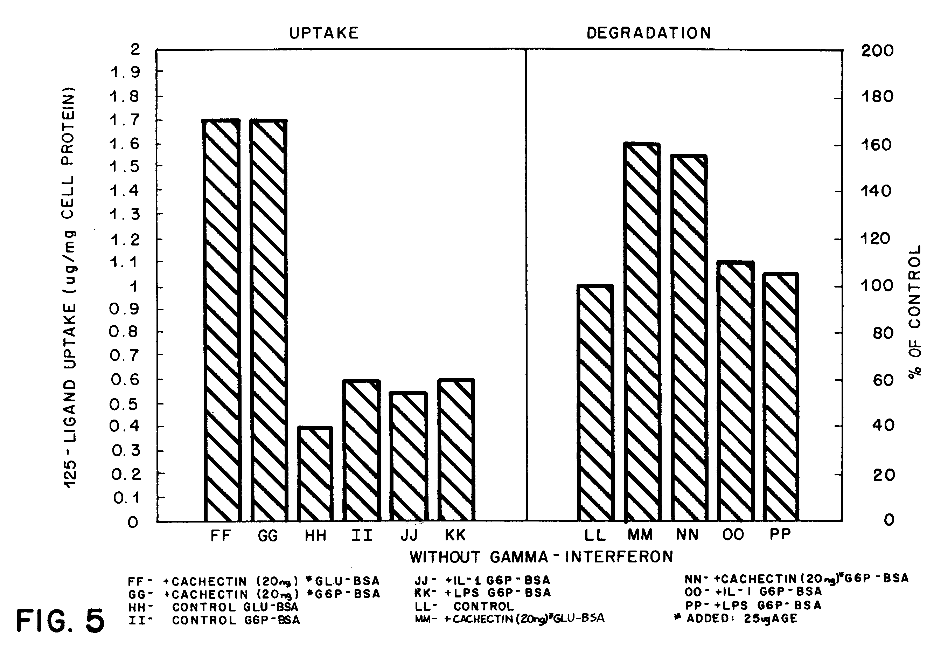

Mouse peritoneal macrophages show increased uptake of AGE-BSA when stimulated with AGE-BSA prepared from glucose-6-phosphate (Figure 5, bar GG). Degradation is also increased (Figure 5, bar NN).

As discussed earlier, phagocytic cells such as macrophages may be stimulated to become activated and remove AGE-macromolecules is after treating the macrophages with a monokine such as with cachectin (synonym = tumor necrosis factor). The structure and properties of cachectin have been previously elucidated by one of the inventors herein. It has been determined that cachectin at a concentration of 20 ng per milliliter of culture fluid stimulates macrophages to express the AGE-receptor and to increase binding, uptake and degradation of AGE-macromolecules. Preincubation of mouse macrophages for 24 hours with 20 ng/ml of cachectin/TNF (without gamma-interferon) followed by incubation with AGE-BSA results in a 4-fold increase in AGE-BSA (made from glucose reacted with albumin) uptake (Figure 5, bar FF). Degradation is also increased by 50% of the control (bar MM).

Similarly, preincubation of 1-day-old human monocytes for 24 to 48 hours with cachectin without gamma-interferon leads to almost doubling of uptake and degradation of AGE-BSA (Figure 6, bar D). Degradation is also increased (bar 1).

The above examples of agents which stimulate macrophages to increase the ability to internalize and degrade AGEs should not be restrictive. Other agents include additional synthetic specific AGEs linked to carrier molecules, other AGEs made from the reaction of sugars with macromolecules, and other monokines which stimulate macrophages to increase their activity toward AGEs.

The method also includes the coadministration of these agents and one or more co-stimulatory agent such as Interleukin-1 and gamma interferon to achieve an even greater stimulation of the body's AGE removal system.

In a further embodiment of the present invention, macrophages could be treated ex vivo with these agents and returned to the body. For example, an extracorporeal blood treatment may be performed in which indwelling lines are placed in a patient's arterial and venous system, and blood is taken from the body, passed through a device and then returned to the patient. The device is contemplated to contain agents which by contact with or exposure to monocytes/macrophages will stimulate them to increase the activity of the AGE removal system. Further, the agents may either be immobilized or may be capable of entering the blood flow. Such an extracorporeal process may be performed alone or jointly with the administration to the patient of other agents to enhance the process, such as the gamma-interferon described above.

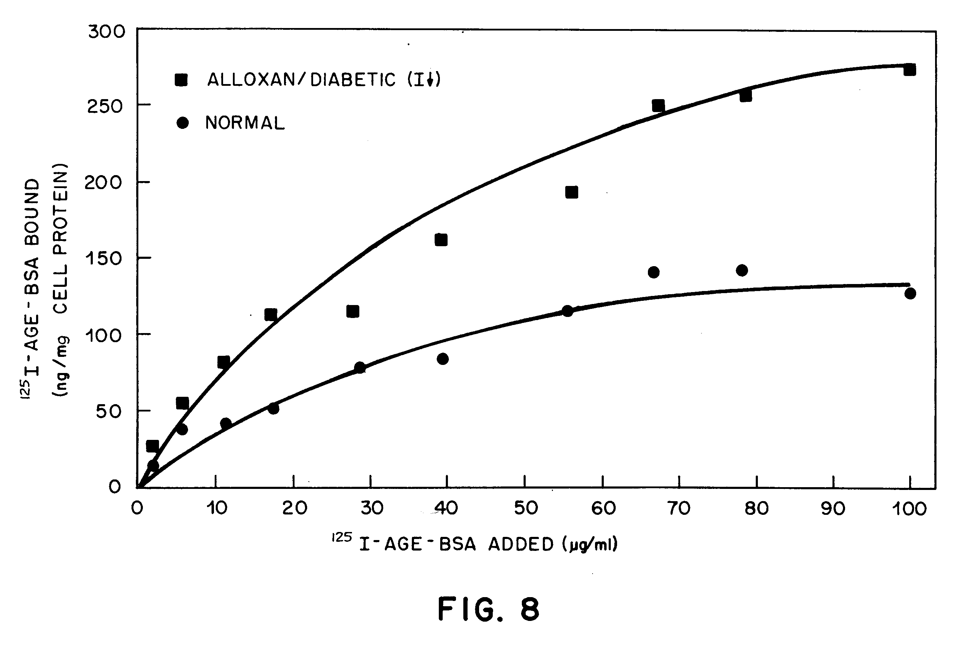

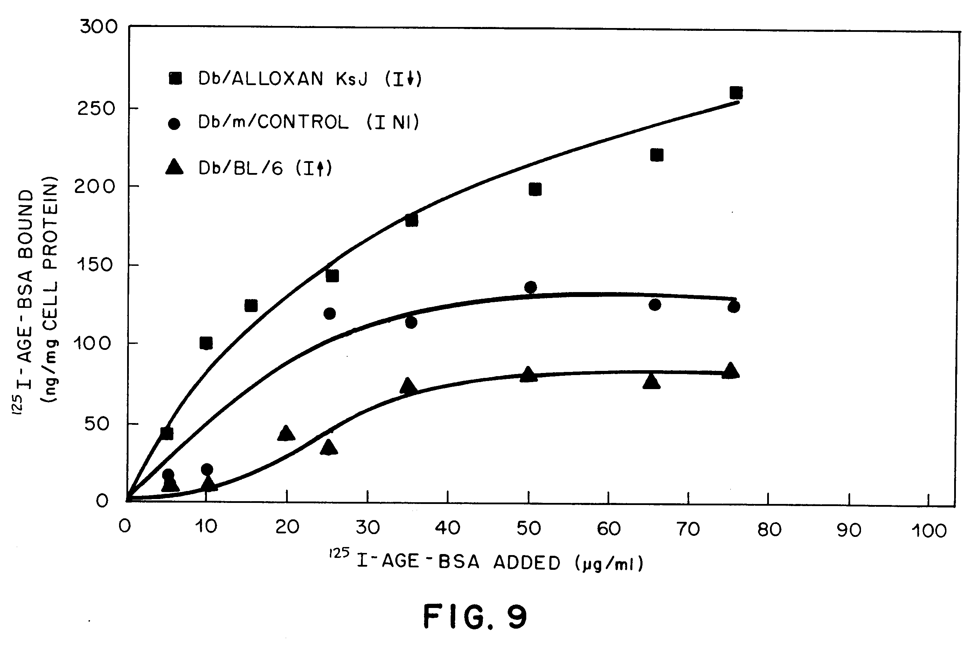

An additional aspect of the present invention herein relates to the observation of the effects of insulin on the macrophage AGE clearance system. It has been found that animals with lower than normal levels of insulin in the blood have an enhanced macrophage AGE clearance system. This has been demonstrated in both experimental animals in which insulin-producing pancreas cells are destroyed by injection of the animal with alloxan, or in genetically diabetic animals which have low insulin levels. In both groups, blood glucose levels are higher than normal. As shown in Figure 8, animals with experimentally-induced diabetes (using alloxan) and resultant low serum insulin levels (25 +_ 6 U/ml) had a two-fold greater activity of binding of AGE-BSA to macrophages as compared with normal animals (serum insulin of 74 +_ 28 U/ml). In Figure 9, it is apparent that C57BL/KsJ, db/db mice, with genetically low insulin levels (8.2 +_ 2 U/ml) have a greater degree of AGE-BSA binding to macrophages than control mice.

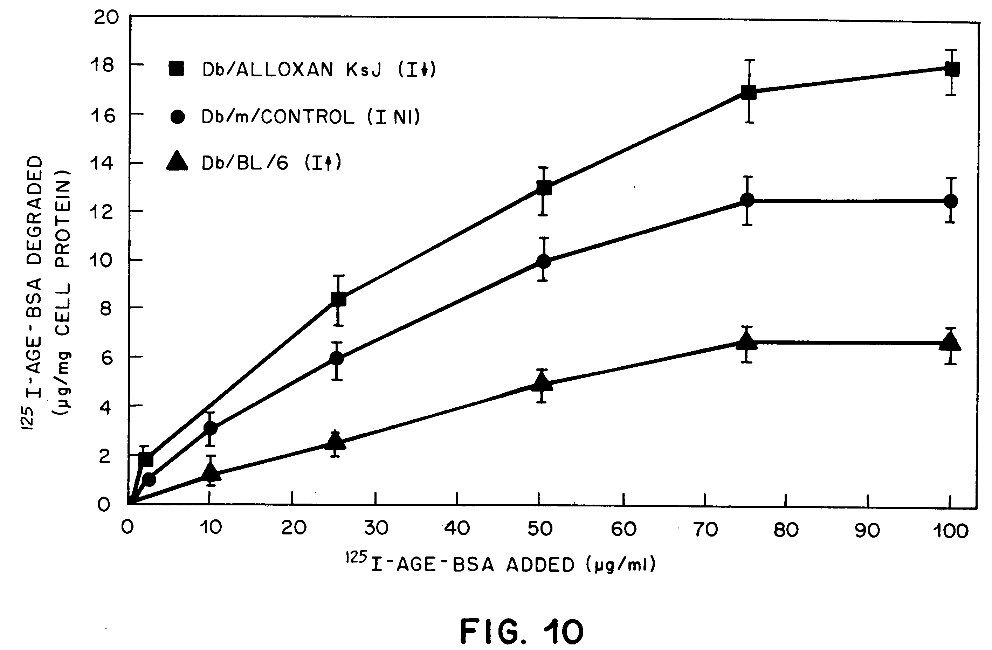

In contrast, animals with high levels of insulin in the blood, such as genetic hyperinsulinaemic animals (C57BL6, db/db, serum insulin > 300 U/ml, Figure 9), have a reduction of about 50% of the activity of binding of AGE-BSA to macrophages compared to normal animals. This suppression of the removal system would have a negative effect since clearance of AGE-macromolecules would be decreased. Degradation of AGE (Figure 10) shows the same relationship to insulin levels.

It is important to note that both the hypoinsulinaemic and hyperinsulinaemic animals had equally abnormal elevations of glucose in the blood.

The effect of insulin on other macrophage receptors is known, however, this is the first report of the role of insulin in regulating the AGE receptor. Because insulin regulates the glucose level in the blood, and glucose is responsible for the production of AGEs which are removed by macrophages, this novel finding suggests an actual correlation between these three phenomena.

An additional observation derived from the present invention, and one which proposes a possible mechanism for the effects of the present method is that macrophages, on stimulation with FFI-carrier proteins or AGE-proteins, will secrete cachectin into the surrounding fluid. Macrophages are known to produce cachectin in response to certain stimuli, but this is the first report of the monokine being produced in response to FFI and AGE-proteins. Since these moieties exist in the body, production of cachectin may occur during recognition, internalization, or degradation of AGEs. Since cachectin is known to increase the removal system, this observation suggests that cachectin may be involved in an amplification phenomenon to signal other macrophages to increase activity of the removal system. The secretion of cachectin by cells exposed to AGEs may also bring about other effects on cells.

Accordingly, one of the therapeutic methods of the present invention comprises providing cachectin or a derivative of cachectin in amounts sufficient to stimulate the removal system. The exact quantities of cachectin to be administered may vary, and the amounts employed in the experiments with cachectin set forth herein are representative. Naturally, specific amounts would be determined by the attending physical or veterinarian administering the treatment.

A further therapeutic application of the present invention lies in the area of immunology. In particular, the recognition and degradation of advanced glycosylation endproducts, such as FFI, by phagocytic cells facilitates the introduction to those cells of certain antigens against which it is desired to raise an immunity. Accordingly, the advanced glycosylation endproduct or FFI could be coupled to an antigen in much the same fashion as disclosed herein for the coupling to any other carrier. Thereafter, the particular phagocytic cells that it is desired to stimulate could be exposed to the coupled complex whereupon the cells would recognize and degrade the latter, and would concomitantly develop specific receptors therefor. These activated phagocytes could then be introduced to the immune system of the animal and would promote the development by the immune system of antibodies to the initial antigen.

The above method may be practiced ex vivo or in vivo. If ex vivo, the method would include the initial removal of the phagocytes from the body and their exposure to the coupled complex to develop their sensitivity to the particular antigen. Thereafter a sample of cells of the animal that is known to be responsible for raising antibodies and that would have been similarly isolated and removed from the animal, or cells from other sources that may perform the same function, would be exposed to the activated phagocytes for a period of time sufficient to enable antibodies to be raised to the antigen of interest. The antibodies could be administered to animals to avert or alleviate the adverse effects of any pathology that might be caused by the invasion of the antigen.

Alternately the ex vivo activated phagocytes could be utilized as a vaccine to inoculate animals against the antigen and to thereby promote the in vivo development of antibodies thereto.

An in vivo protocol contemplates the preparation of the coupled complex between the AGE/FFI and the antigen and the administration of this coupled complex directly to the animal to promote the in vivo activation of phagocytes and the subsequent development of antibodies by the animal's immune system.

A further alternative contemplates the formation of a mixture rather than a coupled complex between the AGE/FFI and the antigen. This mixture could be utilized in place of the coupled complex in the above protocols.

A particular implementation of the ex vivo embodiment of the immunological protocol could include the immobilization of either the coupled complex or the phagocytic cells during all or part of the practice of the particular method. Thus, for example, either the cells or the coupled complex could be immobilized and the unbound material then circulated there past to achieve activation. If it is desired to administer the bound material to the animal, it could be released from the substrate after activation by known techniques.

A further therapeutic protocol is suggested by the foregoing immunological protocols, which is based upon the binding, labeling or other combination of the AGE/FFI in this instance to a particular antigen as defined herein. Thus, the AGE/FFI could be combined with an antibody specific to the particular antigen, and the resulting associated mixture or combined material then placed in contact with the phagocytic cells of the animal/human host to stimulate such cells to recognize and attack the antigen. In such instance, the manner by which contact between the combined material and the phagocytes occurs may vary.

Thereafter, a quantity of the combined material may be introduced in vivo to bind with the target antigen. The stimulated phagocytes would then be introduced or stimulation of the phagocytes would take place concurrently upon the in vivo introduction of the combined material, whereupon the phagocytes would attack and destroy/degrade the complex formed by the combined material and the target antigen, either directly or by means of the secretion of the monokine cachectin/TNF. Regardless of the exact mechanism of final action, the phagocytes would act specifically against the target antigen because of their recognition of the particular AGE/FFI now associated therewith by means of the antibody.
The foregoing therapeutic method may vary as to dosage, manner of administration and periodicity, depending upon the particular host and the attending pathological condition, and is subject to adjustment by the trained physician or veterinarian. This therapy offers a potentially effective avenue of treatment for patients suffering from Acquired Immune Deficiency Syndrome (AIDS) in view of the inability of conventional therapies to alleviate the condition.

All of the above immunological and therapeutic protocols could include the coadministration of one or more of the co-stimulatory agents in the manner set forth earlier herein to potentiate where possible the efficacy of such protocols. The present invention accordingly extends to such variations in its practice.

As mentioned earlier, the present invention contemplates numerous diagnostic applications. In a first application, an assay system may be developed for screening potential drugs effective to act as agents to stimulate the activity of specific phagocytes against advanced glycosylation endproducts. Accordingly, a prospective test drug could be administered to a macrophage sample to determine its stimulatory effect and the macrophage sample after incubation with the test drug could be incubated with a fluid or tissue sample having a quantity of appropriately labeled advanced glycosylation endproducts present therein, so that comparative uptake and degradation studies could then be made. In this assay, plural macrophage colonies could be initially incubated with various of the known agents, so that comparative testing of the prospective drug could take place with greater specificity.

An alternate diagnostic protocol contemplates the investigation of particular phagocytes to determine which of the known agents are most effective in stimulating cellular activity against advanced glycosylation endproducts. Thus, in similar fashion to the protocol described above, plural comparable quantities of a particular phagocytic cell colony could be isolated and incubated with several different known agents, and thereafter incubated with identical corresponding samples containing appropriately labeled advanced glycosylation endproducts, so that a comparison of the extent of activation of the phagocytic cells could then be made, and the agent most effective in stimulating the cellular colony thereby identified. In such manner, the colony exhibiting the greatest uptake and disposal of labeled advanced glycosylation endproducts would correspondingly identify the agent that is most effective.

A further diagnostic utility is predicated upon the study of the various parameters that attend the recognition and removal of advanced glycosylation endproducts and their significance in relation to the operation of animal body systems. In a first embodiment, phagocytic cells such as monocytes and macrophages are removed from the animal's body and are activated ex vivo by exposure to the agents of the present invention. This may be accomplished by an extracorporeal shunt as has been described earlier on herein. After such activation, the phagocytic cells, instead of being returned immediately to the body, are appropriately radiolabeled, as with Technicium, and are then returned to the body, whereupon the animal may undergo radioimaging to note the course of travel of the phagocytes and to thereby determine the location of the concentrations of advanced glycosylation endproducts in the body. In this manner, undesirable concentrations of advanced glycosylation endproducts, such as atheromatous plaques could be discovered, and the clinical significance of these concentrations accordingly assessed. This procedure would provide additional vital information in attempting to assess the pathological state of the animal or patient.

A further diagnostic application likewise seeking to provide vital information regarding pathological status contemplates the preparation of radiolabeled agents and their incubation or contact with phagocytes from a particular animal, and the subsequent measurement of the elapsed time before the labeled agents are recognized and degraded. Such time measurements could be compared against standard measurements determined by testing cells taken from normal animals or patients that were tested under the same protocol. This data could then identify the existence of pathologies such as diabetes, or the others mentioned earlier herein, or other disorders that may adversely affect the operability of the AGE removal system of the body. This diagnostic procedure can be performed in vivo by administering the labeled agents to the animal or patient, or alternatively, could be conducted ex vivo by the isolation of phagocytic cells of the animal or patient outside the body and the incubation of these cells with the labeled agents.

An additional diagnostic technique contemplates a further qualitative analysis of the animal's body. In this procedure particular pathologies may be identified by a method wherein the activity of the phagocytic cells of the animal could be measured and compared against the activities found with normal cells. In such instance, for example, radiolabeled agents could be introduced to the body, or phagocytic cellular colonies from the body could be isolated and incubated with the radiolabeled agents, and the level of activity of the phagocytic cells could then be measured with greater specificity as to particular advanced glycosylation endproducts. This is possible because of the specific nature of the cellular receptors that develop on the phagocytic cells as to particular advanced glycosylation endproducts. Thus, the macrophage or monocytes having greatest involvement with the removal of advanced glycosylation endproducts that would otherwise develop atheromatous plaques, could be isolated and tested for their activity, and if their activity appears to be abnormally increased, the development of such plaques may be indicated. Similar testing protocols could be employed to determine the existence of diabetic aging and the like. Thus, the specific advanced glycosylation endproducts associated with particular conditions would elicit responses from the phagocytes that would suggest any abnormal accumulation of these advanced glycosylation endproducts in the body system. The presence of particular AGEs in the body reflect correspondingly particular pathologies, and the foregoing observation of the phagocytes and the determination of their sensitivity and increased stimulation with respect to particular advanced glycosylation endproducts would suggest the existence of a particular pathology.

## Claims

1. A composition for promoting the sequestration and removal from the body of an animal of target macromolecules that have undergone advanced glycosylation comprising an agent capable of causing the body to increase its activity of recognizing and removing said macromolecules, characterized in that said agent is selected from the group consisting of an advanced glycosylation endproduct, an advanced glycosylation endproduct bound to a carrier, a monokine that stimulates the body to increase said recognizing and removing activity toward said target macromolecule, in combination with a co-stimulatory agent differing from the stimulatory agent.

2. The composition of Claim 1 wherein said advanced glycosylation endproduct comprises the nonenzymatic reaction product of a proteinaceous macromolecule and a sugar.

3. The composition of Claim 2; wherein said advanced glycosylation endproduct is selected from the group consisting of the reaction product of albumin and glucose, the reaction product of albumin and glucose-6-phosphate, the fluorescent chromophore 2-(2-furoyl)-4(5)-(2-furanyl)-1H-imidazole bound to a carrier and mixtures thereof.

4. The composition of Claim 1 wherein said carrier is selected from the group consisting of carbohydrates, proteins, lipids, synthetic polypeptides, biocompatible natural and synthetic resins, antigens, and mixtures thereof.

5. The composition of Claim 1 wherein said co-stimulatory agent is selected from the group consisting of Interleukin-1 and gamma-interferon.

6. The composition of Claim 1, wherein said monokine comprises the polypeptide identified as tumor necrosis factor.

7. The composition of Claim 1 characterized in that it is formulated in order to administer said agent by parenteral means.

8. The composition of Claim 7 wherein said parenteral means comprises injection.

9. The composition of Claim 7 wherein said parenteral means comprises catheterization.

10. The composition of Claim 1 characterized in that it is formulated in order to administer said agent by oral means.

11. The composition of Claim 1 wherein said agent is prepared with a pharmaceutically acceptable carrier.

12. The composition of Claim 1 characterized in that it is formulated in order to allow said agent to be administered both parenterally and extracorporeally to said body fluid.

13. The composition of claim 1 characterized in that it is formulated in order to be administered to a body containing a quantity of insulin, and in order to allow said body to be treated to lower the available active insulin level thereof.

14. A pharmaceutical composition for administration to an animal to promote the sequestration and removal from the animal's body of a target protein that has undergone advanced glycosylation, comprising a pharmaceutically effective amount of an agent capable of causing phagocytic cells in said body to increase their activity of recognizing and removing said target proteins, and a pharmaceutically acceptable carrier, characterized in that the said agent is selected from the group consisting of an advanced glycosylation endproduct bound to a carrier, a monokine that stimulates said phagocytic cells to increase said recognizing and removing activity toward said target proteins, and any of the above optionally in combination with a co-stimulatory agent.

15. The pharmaceutical composition of Claim 6 wherein said advanced glycosylation endproduct comprises the reaction product of a proteinaceous macromolecule and a sugar.

16. The pharmaceutical composition of Claim 6 wherein said advanced glycosylation endproduct is selected from the group consisting of the reaction product of albumin and glucose, the reaction product of albumin and glucose-6-phosphate, the fluorescent chromophore 2-(2-furoyl)-4(5)-(2-furanyl)-1H-imidazole bound to a carrier and mixtures thereof.

17. The pharmaceutical composition of Claim 6 wherein said carrier is selected from the group consisting of carbohydrates, proteins, lipids, synthetic polypeptides, biocompatible natural and synthetic resins, antigens, and mixtures thereof.

18. The pharmaceutical composition of Claim 6 wherein said co-stimulatory agent is selected from the group consisting of Interleukin-1 and gamma-interferon.

19. A method for determining the activity of a material suspected to function as an agent to stimulate a body of an animal to recognize and remove certain macromolecules form the body that have undergone advanced glycosylation, comprising:
preparing a plurality of biological samples derived from the body of said animal containing phagocytic cells therein;
incubating one of said samples with the prospective agent under determination;
incubating all but one of the remainder of said samples with different ones of certain agents known to stimulate said phagocytic cells against advanced glycosylation endproducts, said agent being an advanced glycosylation endproduct, an advanced glycosylation endproduct bound to a carrier, the fluorescent chromophore 2-(2-furoyl)-4(5)-(2-furanyl)-1H-imidazole bound to a carrier, a monokine that stimulates said phagocytic cells to increase said recognizing and removing activity toward said target proteins, any of the above, optionally in combination with a co-stimulatory agent, or a mixture thereof;
maintaining the last of said biological samples as a control;
introducing each of said samples to an individual aliquot quantity of a known advanced glycosylation endproduct having a suitable label associated therewith;
incubating each of the samples for up to about 24 hours; and
observing the samples thereafter to measure the uptake and degradation of said labeled advanced glycosylation endproduct, and comparing the activity of the cells incubated with said prospective agent under determination with that of the samples incubated with known agents, to determine the presence and extent of stimulating activity of said prospective agent.

20. A method for preparing a composition for treating an animal having a pathology caused at least in part by the accumulation in the body of the animal of macromolecules that have undergone advanced glycosylation, comprising isolating a quantity of cells from said animal, growing an additional quantity of said phagocytic cells from said isolated phagocytic cells in a nutrient medium free from insulin, harvesting the phagocytic cells so grown, and introducing them into a pharmaceutically acceptable carrier able to be introduced into said animal's body.

21. A method for obtaining activated cells capable of averting the adverse sequelae of the accumulation of advanced glycosylation endproducts in the body of an animal, comprising:
isolating a quantity of phagocytic cells from said animal; and
treating the phagocytic cells so isolated with an agent capable of causing said phagocytic cells to increase their activity of recognizing and removing macromolecules that have undergone advanced glycosylation.

22. A method for preparing a composition able to immunize an animal against a particular antigen comprising:
preparing a mixture of said antigen and an agent capable of causing phagocytic cells in the body of said animal to increase their activity of recognizing and removing macromolecules that have undergone advanced glycosylation; and
obtaining said mixture in a form administrable to the animal to stimulate the development by the animal's immune system of antibodies to said antigen.

23. A method for preparing activated phagocytic cells able to be introduced into an animal's body to immunize the animal against a particular antigen promoting in vivo development of antibodies to said antigen, said method comprising;
isolating a quantity of phagocytic cells from said animal;
preparing a mixture of said antigen and an agent capable of causing said phagocytic cells to increase their activity of recognizing and removing macromolecules that have undergone advanced glycosylation; and
incubating the phagocytic cells so isolated with said mixture to activate said phagocytic cells there against.

24. A method for preparing a composition able to immunize an animal against a particular antigen by stimulating development of antibodies to said antigen by the animal's immune system, said method comprising the step of preparing a coupled complex between said antigen and an agent capable of causing phagocytic cells in the body of said animal to increase their activity of recognizing and removing macromoleclules that have undergone advanced glycosylation.

25. A method for obtaining activated phagocytic cells adapted to be introduced into an animal's body for immunizing the animal against a particular antigen comprising;
isolating a quantity of phagocytic cells from said animal;
preparing a coupled complex between said antigen and an agent capable of causing said phagocytic cells to increase their activity of recognizing and removing macromolecules that have undergone advanced glycosylation; and
incubating the phagocytic cells so isolated with said coupled complex to activate said phagocytic cells thereagainst.

26. The method of Claim 23 or 25 wherein prior to being incubated with said mixture or complex, said cells are caused to become affixed to an immobilized substrate, and subsequent to being incubated with said mixture or complex, said cells are released from said substrate.

27. A method for obtaining cellular material able to immunize an animal against a particular antigen, negating said antigen, the method comprising:
isolating a quantity of phagocytic cells from said animal;
preparing a coupled complex between said antigen and an agent capable of causing said phagocytic cells to increase their activity of recognizing and removing macromolecules that have undergone advanced glycosylation;
incubating the phagocytic cells so isolated with said coupled complex to activate said phagocytic cells thereagainst; and
isolating cellular material from said animal that participates in the development of the animal's immune response and incubating said activated phagocytic cells therewith for a period of time and under conditions sufficient to cause said cellular material to develop antibodies to said antigen.

28. The method of Claim 27 wherein said coupled complex is affixed to an immobilized substrate.

29. The method of Claim 27 wherein said cells are affixed to an immobilized substrate.

30. The method of Claim 24, 25 or 27 wherein said agent is an advanced glycosylation endproduct, an advanced glycosylation endproduct bound to a carrier, the fluorescent chromophore 2-(2-furoyl)-4(5)-(2-furanyl)-1H-imidazole bound to a carrier, or a mixture thereof.

31. The method of Claim 24, 25 or 27 wherein a monokine that stimulates the body to increase said recognizing and removing activity toward said target macromolecule is included for co-administration with said coupled complex, said activated phagocytic cells or said cellular material.

32. The method of Claim 24, 25 or 27 wherein a co-stimulatory agent is included for co-administration with said coupled complex, said activated phagocytic cells or said cellular matter.

33. A method for obtaining means for identifying and measuring the extent of pathological conditions in an animal, comprising:
isolating a quantity of phagocytic cells from said animal;
attaching to said cells a radioactive label so that when said labeled cells are introduced into said animal's body, said labeled cells are able to be traced to determine the location and extent of accumulation of advanced glycosylation endproducts, and to thereby determine the corresponding location and extent of pathologies having such accumulations as a manifestation thereof.

34. The method of Claim 33 wherein prior to said introducing step said phagocytic cells are incubated with an agent capable of causing said phagocytic cells to increase their activity of recognizing and removing macromolecules that have undergone advanced glycosylation.

35. A method for preparing means for measuring the pathological status of an animal comprising:
preparing an agent capable of causing phagocytic cells of said animal to increase their activity of recognizing and removing macromolecules that have undergone nonenzymatic advanced glycosylation;
attaching an appropriate label to said agent; sop that when said labeled agent is administered to said animal that pathological status being shown by measuring the time required by said body to recognize and remove said labeled agent and by comparing the time results received with the time results observed from animals in a normal state.

36. A method for measuring the pathological status of an animal comprising:
preparing an agent capable of causing phagocytic cells of said animal to increase their activity of recognizing and removing macromolecules that have undergone nonenzymatic advanced glycosylation;
attaching an appropriate label to said agent;
isolating a sample of phagocytic cells from said animal and incubating said cells with said labeled agent;
measuring the time required by said sample to recognize and remove said labeled agent; and
comparing the time results received with time results observed from cellular samples of animals in a normal state.

37. A method for measuring the identity and extent of advanced glycosylation endproducts in a cellular sample from an animal's body comprising:
isolating a colony of phagocytic cells from said body;
incubating said colony with an advanced glycosylation endproduct having a label attached thereto;
measuring the uptake and/or degradation of the said labeled advanced glycosylation endproduct by said colony;
comparing the results of said measurement with results derived from identical measurements taken from cells under normal activation; and
determining therefrom the extent of activation of the cells of said colony by advanced glycosylation endproducts and thereby, the amount and identity of advanced glycosylation endproducts present in said cellular sample.

38. The method of Claims 21, 22, 23 or 27, wherein said agent is an advanced glycosylation endproduct, an advanced glycosylation endproduct bound to a carrier, the fluorescent chromophore 2-(2-furoyl)-4(5)-(2-furanyl)-1H-imidazole bound to a carrier, a monokine that stimulates the body to increase said recognizing and removing activity toward said target macromolecules, any of the above in combination with a co-stimulatory agent, or a mixture thereof.

39. The method of Claim 21, wherein said cells are treated in such a manner to be able to be re-introduced to said body by parenteral means.

40. The method of Claim 39, wherein said parental means comprises injection.

41. The method of Claim 39, wherein said parental means comprises catheterization.

42. Use of a composition comprising an associated mixture of an antibody to target proteins of cells, and an agent capable of causing phagocytic cells in an animal to increase their activity for recognizing and removing macromolecules that have undergone advanced glycosylation, for the preparation of a mixture for treating animal pathology with concomitant debilitation of certain target proteins or cells, by administration of said mixture to the animal to form a complex between the said mixture and said target proteins or cells and stimulation of the phagocytic cells of the animal to increase the specific recognition and removal of said mixture, so that said phagocytes act to recognize and remove said mixture and thereby cause the destruction of said target proteins or cells.

43. The use of Claim 42, wherein said agent is selected from the group consisting of an advanced glycosylation endproduct, an advanced glycosylation endproduct bound to a carrier, a monokine that stimulates the body to increase said recognizing and removing activity toward said target macromolecules, and of the above in combination with a co-stimulatory agent, and mixtures thereof.

44. The use of Claim 43, wherein said advanced glycosylation endproduct comprises the reaction product of a proteinaceous macromolecule and a sugar.

45. The method of Claim 43 wherein said advanced glycosylation endproduct is selected from the group consisting of the reaction product of albumin and glucose, the reaction product of albumin and glucose-6-phosphate, the fluorescent chromophore 2-(2-furoyl)-4(5)-(2-furanyl)-1H-imidasole bound to a carrier, and mixtures thereof.

46. The use of Claim 43, wherein said carrier is selected from the group consisting of carbohydrates, proteins, lipids, synthetic polypeptides, biocompatible natural and synthetic resins, antigens, and mixtures thereof.

47. The use of Claim 43, wherein said monokine comprises the polypeptide identified as tumor necrosis factor.

48. The use of Claim 43, wherein said co-stimulatory agent is selected from the group consisting of Interleukin-1 and gamma-interferon.

## Patentansprüche

1. Eine Zusammensetzung zur Förderung der Abtrennung und der Entfernung von Zielmakromolekülen, die einer fortgeschrittenen Glykosylierung unterworfen wurden, aus dem Körper eines Tieres, umfassend ein Mittel, das geeignet ist, den Körper zu veranlassen, seine Aktivität in bezug auf das Erkennen und Entfernen der Makromoleküle zu steigern, gekennzeichnet dadurch, daß das Mittel aus der Gruppe ausgewählt wird, die besteht aus einem Endprodukt der fortgeschrittenen Glykosylierung, einem Endprodukt der fortgeschritten Glykosylierung, das an einen Träger gebundenen ist, einem Monokin, das den Körper stimuliert, die Erkennungs- und Entfernungsaktivität gegen das Makromolekül zu steigern, in Verbindung mit einem costimulatorischen Mittel, das von dem stimulatorischen Mittel verschieden ist.

2. Die Zusammensetzung gemäß Anspruch 1, wobei das Endprodukt der fortgeschrittenen Glykosylierung das nichtenzymatische Reaktionsprodukt eines proteinhaltigen Makromoleküls und eines Zuckers umfaßt.

3. Die Zusammensetzung gemäß Anspruch 2, wobei das Endprodukt der fortgeschrittenen Glykosylierung aus der Gruppe ausgewählt wird, die besteht aus dem Reaktionsprodukt von Albumin und Glukose, dem Reaktionsprodukt von Albumin und Glukose-6-Phosphat, dem fluoreszierenden Chromophor 2-(2-Furoyl)-4(5)-(2-furanyl)-1H-imidazol, das an einen Träger gebunden ist, und Mischungen davon.

4. Die Zusammensetzung gemäß Anspruch 1, wobei der Träger aus der Gruppe ausgewählt wird, die aus Kohlenhydraten, Proteinen, Lipiden, synthetischen Polypeptiden, biokompatiblen natürlichen und synthetischen Harzen, Antigenen und Mischungen davon besteht.

5. Die Zusammensetzung gemäß Anspruch 1, wobei das costimulatorische Mittel aus der Gruppe ausgewählt wird, die aus Interleukin-1 und gamma-Interferon besteht.

6. Die Zusammensetzung gemäß Anspruch 1, wobei das Monokin das als Tumornekrosefaktor identifizierte Polypeptid umfaßt.

7. Die Zusammensetzung gemäß Anspruch 1, gekennzeichnet dadurch, daß diese formuliert ist, um das Mittel über parenterale Mittel zu verabreichen.

8. Die Zusammensetzung gemäß Anspruch 7, wobei die parenteralen Mittel die Injektion umfassen.

9. Die Zusammensetzung gemäß Anspruch 7, wobei die parenteralen Mittel die Katheterisierung umfassen.

10. Die Zusammensetzung gemäß Anspruch 1, gekennzeichnet dadurch, daß diese formuliert ist, um das Mittel über orale Mittel zu verabreichen.

11. Die Zusammensetzung gemäß Anspruch 1, wobei das Mittel mit einem pharmazeutisch annehmbaren Träger hergestellt ist.

12. Die Zusammensetzung gemäß Anspruch 1, gekennzeichnet dadurch, daß diese formuliert ist, um das Mittel sowohl parenteral als auch extrakorporal der Körperflüssigkeit zuführen zu lassen.

13. Die Zusammensetzung gemäß Anspruch 1, gekennzeichnet dadurch, daß diese formuliert ist, um einem Körper, der eine Insulinmenge enthält, verabreicht zu werden, und um es dem Körper, der behandelt wird, zu ermöglichen, den verfügbaren aktiven Insulinspiegel davon zu erniedrigen.

14. Eine pharmazeutische Zusammensetzung zur Verabreichung an ein Tier, um die Abtrennung und Entfernung eines Zielproteines, das einer fortgeschrittenen Glykosylierung unterworfen wurde, aus dem Körper des Tieres zu fördern, umfassend eine pharmazeutisch wirksame Menge eines Mittels, das geeignet ist, phagozytierende Zellen in dem Körper zu veranlassen, ihre Aktivität des Erkennens und Entfernens der Zielproteine zu steigern, und einen pharmazeutisch annehmbaren Träger, gekennzeichnet dadurch, daß das Mittel aus der Gruppe ausgewählt wird, die besteht aus einem Endprodukt der fortgeschrittenen Glykosylierung, das an einen Träger gebunden ist, einem Monokin, das die phagozytischen Zellen stimuliert, um die Erkennungs- und Entfernungsaktivität gegen die Zielproteine zu steigern, und optional jedes der obigen in Kombination mit einem costimulatorischen Mittel.

15. Die pharmazeutische Zusammensetzung gemäß Anspruch 6, wobei das Endprodukt der fortgeschrittenen Glykosylierung das Reaktionsprodukt eines proteinhaltigen Makromoleküles und eines Zuckers umfaßt.

16. Die pharmazeutische Zusammensetzung gemäß Anspruch 6, wobei das Endprodukt der fortgeschrittenen Glykosylierung aus der Gruppe ausgewählt wird, die besteht aus dem Reaktionsprodukt von Albumin und Glukose, dem Reaktionsprodukt von Albumin und Glukose-6-phosphat, dem fluoreszierenden Chromophor 2-(2-Furoyl)-4-(5)-(2-furanyl)-1H-imidazol, das an einen Träger gebunden ist, und Mischungen davon.

17. Die pharmazeutische Zusammensetzung gemäß Anspruch 6, wobei der Träger aus der Gruppe ausgewählt wird, die aus Kohlenhydraten, Proteinen, Lipiden, synthetischen Polypeptiden, biokompatiblen natürlichen und synthetischen Harzen, Antigenen, und Mischungen davon besteht.

18. Die pharmazeutische Zusammensetzung gemäß Anspruch 6, wobei das costimulatorische Mittel aus der Gruppe ausgewählt wird, die aus Interleukin-1 und gamma-Interferon besteht.

19. Ein Verfahren zur Bestimmung der Aktivität eines Materials, von dem vermutet wird, daß es als ein Mittel wirkt, den Körper eines Tieres zu stimulieren, um bestimmte Makromoleküle, die einer fortgeschrittenen Glykosylierung unterworfen wurden, zu erkennen und aus dem Körper zu entfernen, umfassend:
das Herstellen einer Vielzahl von biologischen Proben, die aus dem Körper des Tieres stammen, die darin phagozytische Zellen enthalten;
das Inkubieren einer der Proben mit dem voraussichtlichen Mittel unter Beobachtung;
das Inkubieren aller bis auf einen der Probenreste mit verschiedenen bestimmten Mitteln, von denen bekannt ist, die phagozytischen Zellen gegen fortschrittene Glykosylierungsendprodukte zu stimulieren, wobei das Mittel ein Endprodukt der fortgeschrittenen Glykosylierung, ein Endprodukt der fortgeschrittenen Glykosylierung, das an einen Träger gebunden ist, das fluoreszierende Chromophor 2-(2-Furoyl)-4(5)-2(2-furanyl)-1H-imidazol, das an einen Träger gebunden ist, ein Monokin, das die phagozytischen Zellen stimuliert, die Erkennungs- und Entfernungsaktivität gegen die Zielproteine zu steigern, jedes der obigen optional in Verbindung mit einem costimulatorischen Mittel oder eine Mischung davon ist;
das Halten der letzten der biologischen Proben als eine Kontrolle;
das Einbringen jeder der Proben in eine individuelle Teilmenge eines bekannten Endprodukt der fortgeschrittenen Glykosylierung, das eine damit verbundene geeignete Markierung hat;
das Inkubieren jeder dieser Proben für bis zu etwa 24 Stunden; und
das Untersuchen der Proben danach, um die Aufnahme und den Abbau von dem markierten Endprodukt der fortgeschrittenen Glykosylierung zu messen, und Vergleichen der Aktivität der Zellen, die mit dem voraussichtlichen Mittel unter Beobachtung inkubiert wurden, mit denen der Proben, die mit bekannten Mitteln inkubiert wurden, um das Vorhandensein und das Ausmaß der stimulierenden Aktivität des voraussichtlichen Mittels zu bestimmen.

20. Ein Verfahren zur Herstellung einer Zusammensetzung zur Behandlung eines Tieres mit einem Krankheitsbild, das wenigstens zum Teil durch die Ansammlung von Makromolekülen, die einer fortgeschrittenen Glykosylierung unterworfen wurden, in dem Körper des Tieres verursacht wurde, umfassend die Isolierung einer Menge an Zellen aus dem Tier, das Wachsenlassen einer zusätzlichen Menge der phagozytischen Zellen von den isolierten phagozytischen Zellen in einem Nährstoffmedium, das frei von Insulin ist, dem Ernten der so gezogenen phagozytischen Zellen, und das Einbringen von diesen in einen pharmazeutisch annehmbaren Träger, der geeignet ist, in den Tierkörper eingebracht zu werden.

21. Ein Verfahren zur Gewinnung von aktivierten Zellen, die geeignet sind, die nachteiligen Folgen der Ansammlung von fortgeschrittenen Glykosylierungsendprodukten in dem Körper eines Tieres zu verhüten, umfassend:
das Isolieren einer Menge an phagozytischen Zellen aus dem Tier; und
das Behandeln der so isolierten phagozytischen Zellen mit einem Mittel, das geeignet ist, die phagozytischen Zellen zu veranlassen, ihre Aktivität des Erkennens und Entfernens von Makromolekülen, die einer fortgeschrittenen Glykosylierung unterworfen wurden, zu steigern.

22. Ein Verfahren zur Herstellung einer Zusammensetzung, die geeignet ist, ein Tier gegen ein besonderes Antigen zu immunisieren, umfassend:
das Herstellen einer Mischung des Antigens und eines Mittels, das geeignet ist, phagozytische Zellen in dem Körper des Tieres zu veranlassen, ihre Aktivität des Erkennens und Entfernens von Makromolekülen, die einer fortgeschrittenen Glykosylierung unterworfen wurden, zu steigern; und
das Gewinnen der Mischung in einer Form, die dem Tier verabreichbar ist, um die Entwicklung von Antikörpern gegen das Antigen durch das Immunsystem des Tieres zu stimulieren.

23. Ein Verfahren zur Herstellung von aktivierten phagozytischen Zellen, die geeignet sind, in den Körper eines Tieres eingebracht zu werden, um das Tier gegen ein besonderes Antigen, das in vivo die Entwicklung von Antikörpern gegen das Antigen unterstützt, zu immunisieren, wobei das Verfahren umfaßt;
das Isolieren einer Menge an phagozytischen Zellen aus dem Tier;
das Herstellen einer Mischung des Antigens und eines Mittels, das geeignet ist, die phagozytischen Zellen zu veranlassen, ihre Aktivität des Erkennens und Entfernens von Makromolekülen, die einer fortgeschrittenen Glykosylierung unterworfen wurden, zu steigern; und
das Inkubieren der so isolierten phagozytischen Zellen mit der Mischung, um die phagozytischen Zellen dagegen zu aktivieren.

24. Ein Verfahren zur Herstellung einer Zusammensetzung, die geeignet ist, ein Tier gegen ein besonderes Antigen zu immunisieren, indem man die Entwicklung von Antikörpern gegen das Antigen durch das Immunsystem des Tieres stimuliert, wobei das Verfahren den Schritt der Herstellung eines gekoppelten Komplexes zwischen dem Antigen und einem Mittel, das geeignet ist, phagozytische Zellen in dem Körper des Tieres zu veranlassen, ihre Aktivität des Erkennens und Entfernens von Makromolekülen, die einer fortgeschrittenen Glykosylierung unterworfen wurden, zu steigern, umfaßt.

25. Ein Verfahren zur Gewinnung von aktivierten phagozytischen Zellen, die angepaßt sind, in den Körper eines Tieres zum Immunisieren des Tieres gegen ein besonderes Antigen eingebracht zu werden, umfassend;
das Isolieren einer Menge an phagozytischen Zellen aus dem Tier;
das Herstellen eines gekoppelten Komplexes zwischen dem Antigen und einem Mittel, das geeignet ist, phagozytische Zellen zu veranlassen, ihre Aktivität des Erkennens und Entfernens von Makromolekülen, die einer fortgeschrittenen Glykosylierung unterworfen wurden, zu steigern; und
das Inkubieren der so isolierten phagozytischen Zellen mit dem gekoppelten Komplex, um die phagozytischen Zellen dagegen zu aktivieren.

26. Das Verfahren gemäß Anspruch 23 oder 25, wobei man bewirkt, daß man die Zellen, bevor sie mit der Mischung oder dem Komplex inkubiert werden, an einem immobilisierten Träger binden läßt, und, nachdem sie mit der Mischung oder dem Komplex inkubiert wurden, die Zellen von dem Träger freigesetzt werden.

27. Ein Verfahren zum Erhalten von zellulärem Material, das geeignet ist, ein Tier gegen ein besonderes Antigen zu immunisieren, wobei man das Antigen vernichtet, wobei das Verfahren umfaßt:
das Isolieren einer Menge an phagozytischen Zellen aus dem Tier;
das Herstellen eines gekoppelten Komplexes zwischen dem Antigen und einem Mittel, das geeignet ist, die phagozytischen Zellen zu veranlassen, ihre Aktivität des Erkennens und Entfernens von Makromolekülen, die einer fortgeschrittenen Glykosylierung unterworfen wurden, zu steigern;
das Inkubieren der so isolierten phagozytischen Zellen mit dem gekoppelten Komplex, um die phagozytischen Zellen dagegen zu aktivieren; und
das Isolieren von zellulärem Material aus dem Tier, das bei der Entwicklung der Immunantwort des Tieres teilnimmt, und Inkubieren der damit aktivierten phagozytischen Zellen für einen Zeitraum und unter Bedingungen, die ausreichend sind, das zelluläre Material zu veranlassen, Antikörper gegen das Antigen zu entwickeln.

28. Das Verfahren gemäß Anspruch 27, wobei der gekoppelte Komplex an einen immobilisierten Träger gebunden ist.

29. Das Verfahren gemäß Anspruch 27, wobei die Zellen an einen immobilisierten Träger gebunden sind.

30. Das Verfahren gemäß Anspruch 24, 25 oder 27, wobei das Mittel ein Endprodukt der fortgeschrittenen Glykosylierung, ein Endprodukt der fortgeschrittenen Glykosylierung, das an einen Träger gebunden ist, das fluoreszierende Chromophor 2-(2-Furoyl)-4(5)-(2-furanyl)-1H-imidazol, das an einen Träger gebunden ist, oder eine Mischung davon ist.

31. Das Verfahren gemäß Anspruch 24, 25 oder 27, wobei ein Monokin, das den Körper stimuliert, um die Erkennungs- und Entfernungsaktivität gegen das Zielmakromolekül zu steigern, zur Mitverabreichung mit dem gekoppelten Komplex, den aktivierten phagozytischen Zellen oder dem zellulären Material beinhaltet ist.

32. Das Verfahren gemäß Anspruch 24, 25 oder 27, wobei ein costimulatorisches Mittel für die Mitverabreichung mit dem gekoppelten Komplex, den aktivierten phagozytischen Zellen oder dem zellulären Material eingeschlossen ist.

33. Ein Verfahren zur Gewinnung von Mitteln zum Identifizieren und Vermessen des Ausmaßes von pathologischen Zuständen in einem Tier, umfassend:
das Isolieren einer Menge an phagozytischen Zellen aus dem Tier;
das Anheften einer radioaktiven Markierung an die Zellen, so daß, wenn die markierten Zellen in den Körper des Tieres eingebracht werden, die markierten Zellen nachgewiesen werden können, um den Ort und das Ausmaß der Ansammlung von Endprodukten der fortgeschrittenen Glykosylierung zu bestimmen, und um dadurch den entsprechenden Ort und das Ausmaß von Krankheitsbildern mit solchen Ansammlungen als eine Erscheinungsform davon zu bestimmen.

34. Das Verfahren gemäß Anspruch 33, wobei vor dem Einbringungsschritt die phagozytischen Zellen mit einem Mittel, das geeignet ist, die phagozytischen Zellen zu veranlassen, ihre Aktivität des Erkennens und Entfernens von Makromolekülen, die einer fortgeschrittenen Glykosylierung unterworfen wurden, zu steigern, inkubiert werden.

35. Ein Verfahren zur Herstellung von Mitteln zur Vermessung des pathologischen Zustandes eines Tieres, umfassend:
das Herstellen eines Mittels, das geeignet ist, phagozytische Zellen des Tieres zu veranlassen, ihre Aktivität des Erkennnens und Entfernens von Makromolekülen, die einer nichtenzymatischen fortgeschrittenen Glykosylierung unterworfen wurden, zu steigern;
das Anbringen einer geeigneten Markierung an dem Mittel, so daß, wenn das markierte Mittel dem Tier verabreicht wird, der pathologische Zustand durch Messen der Zeit, die der Körper benötigt, um das markierte Mittel zu erkennen und zu entfernen, und durch Vergleichen der erhaltenen Zeitergebnisse mit den Zeitergebnissen, die bei Tieren in einem normalen Zustand beobachtet werden, angezeigt wird.

36. Ein Verfahren zum Bestimmen des pathologischen Zustandes eines Tieres, umfassend:
das Herstellen eines Mittels, das geeignet ist, phagozytische Zellen des Tieres zu veranlassen, ihre Aktivität des Erkennens und Entfernens von Makromolekülen, die einer nichtenzymatischen fortgeschrittenen Glykosylierung unterworfen wurden, zu steigern;
das Anbringen einer geeigneten Markierung an dem Mittel;
das Isolieren einer Probe von phagozytischen Zellen aus dem Tier und Inkubieren der Zellen mit dem markierten Mittel;
das Bestimmen der Zeit, die von der Probe benötigt wird, um das markierte Mittel zu erkennen und zu entfernen; und
das Vergleichen der erhaltenen Zeitergebnisse mit den Zeitergebnissen, die bei zellulären Proben von Tieren in einem normalen Zustand beobachtet wurden.

37. Ein Verfahren zum Vermessen der Identität und des Ausmaßes an fortgeschrittenen Glykosylierungsendprodukten in einer zellulären Probe aus einem Tierkörper, umfassend:
das Isolieren einer Kolonie von phagozytischen Zellen aus dem Körper;
das Inkubieren der Kolonie mit einem fortgeschrittenen Glykosylierungsendprodukt, das eine daran gebundene Markierung besitzt;
das Messen der Aufnahme und/oder des Abbaus von dem markierten Endprodukt der fortgeschrittenen Glykosylierung durch die Kolonie;
das Vergleichen der Ergebnisse der Messung mit den Ergebnissen, die von identischen Messungen, die von Zellen bei normaler Aktivierung bestimmt wurden, stammen; und
daraus das Bestimmen des Ausmaßes der Aktivierung der Zellen der Kolonie durch die Endprodukte der fortgeschrittenen Glykosylierung und damit die Menge und Identität der Endprodukte der fortgeschrittenen Glykosylierung, die in der zellulären Probe vorhanden sind.

38. Das Verfahren gemäß den Ansprüchen 21, 22, 23 oder 27, wobei das Mittel ein Endprodukt der fortgeschrittenen Glykosylierung, ein Endprodukt der fortgeschrittenen Glykosylierung, das an einen Träger gebunden ist, das fluoreszierende Chromophor 2-(2-Furoyl)-4(5)-(2-furanyl)-1H-imidazol, das an einen Träger gebunden ist, ein Monokin, das den Körper stimuliert, um die Erkennungs- und Entfernungsaktivität gegen die Zielmakromoleküle zu steigern, jedes der obigen in Kombination mit einem costimulatorischen Mittel, oder eine Mischung davon ist.

39. Das Verfahren gemäß Anspruch 21, wobei die Zellen auf eine solche Art behandelt werden, daß sie in den Körper über parenterale Mittel wieder eingebracht werden können.

40. Das Verfahren gemäß Anspruch 39, wobei die parenteralen Mittel die Injektion umfaßt.

41. Das Verfahren gemäß Anspruch 39, wobei die parenteralen Mittel die Katheterisierung umfaßt.

42. Verwendung einer Zusammensetzung, umfassend eine assoziierte Mischung eines Antikörpers an Zielproteine von Zellen, und ein Mittel, das geeignet ist, phagozytische Zellen in einem Tier zu veranlassen, ihre Aktivität des Erkennens und Entfernens von Makromolekülen, die einer fortgeschrittenen Glykosylierung unterworfen wurden, zu steigern, für die Herstellung einer Mischung zum Behandeln eines Krankheitsbildes eines Tieres mit gleichzeitiger Schwächung von bestimmten Zielproteinen oder Zellen, durch Verabreichung der Mischung an das Tier, um einen Komplex zwischen der Mischung und den Zielproteinen oder Zellen zu bilden, und Stimulation der phagozytischen Zellen des Tieres, um die spezifische Erkennung und Entfernung der Mischung zu steigern, so daR die Phagozyten wirken, um die Mischung zu erkennen und zu entfernen, und dabei den Abbau der Zielproteine oder Zellen bewirken.

43. Die Verwendung gemäß Anspruch 42, wobei das Mittel aus der Gruppe ausgewählt wird, die besteht aus einem Endprodukt der fortgeschrittenen Glykosylierung, einem Endprodukt der fortgeschrittenen Glykosylierung, das an einen Träger gebunden ist, einem Monokin, das den Körper stimuliert, die Erkennungs- und Entfernungsaktivität gegen die Zielmoleküle zu steigern, und die obigen in Kombination mit einem costimulatorischen Mittel, und Mischungen davon.

44. Die Verwendung gemäß Anspruch 43, wobei das Endprodukt der fortgeschrittenen Glykosylierung das Reaktionsprodukt eines proteinhaltigen Makromoleküles und eines Zuckers umfaßt.

45. Das Verfahren gemäß Anspruch 43, wobei das Endprodukt der fortgeschrittenen Glykosylierung aus der Gruppe ausgewählt wird, die besteht aus dem Reaktionsprodukt von Albumin und Glukose, dem Reaktionsprodukt von Albumin und Glukose-6-phosphat, dem fluoreszierenden Chromophor 2-(2-Furoyl)-4(5)-(2-furanyl)-1H-imidazol, das an einen Träger gebunden ist, und Mischungen davon.

46. Die Verwendung gemäß Anspruch 43, wobei der Träger aus der Gruppe ausgewählt wird, die aus Kohlenhydraten, Proteinen, Lipiden, synthetischen Polypeptiden, biokompatiblen natürlichen und synthetischen Harzen, Antigenen und Mischungen davon besteht.

47. Die Verwendung gemäß Anspruch 43, wobei das Monokin das als Tumornekrosefaktor identifizierte Polypeptid umfaßt.

48. Die Verwendung gemäß Anspruch 43, wobei das costimulatorische Mittel aus der Gruppe ausgewählt wird, die aus Interleukin-1 und gamma-Interferon besteht.

## Revendications

1. Composition pour favoriser la séquestration et l'élimination, de l'organisme d'un animal, de macromolécules cibles ayant subi une glycosylation avancée comprenant un agent capable de faire en sorte que l'organisme augmente son activité de reconnaissance et d'élimination desdites macromolécules, caractérisée en ce que ledit agent est choisi dans le groupe constitué par un produit terminal de glycosylation avancée, un produit terminal de glycosylation avancée lié à un vecteur, une monokine qui stimule l'organisme afin d'augmenter ladite activité de reconnaissance et d'élimination vers ladite macromolécule cible, en combinaison avec un agent co-stimulateur qui diffère de l'agent stimulateur.

2. Composition selon la revendication 1, dans laquelle ledit produit termina de glycosylation avancée comprend le produit de la réaction non enzymatique d'une macromolécule protéinée et d'un sucre.

3. Composition selon la revendication 2, dans laquelle ledit produit terminal de glycosylation avancée est choisi dans le groupe constitué par le produit de la réaction de l'albumine et du glucose, le produit de la réaction de l'albumine et du glucose-6-phosphate, le chromophore fluorescent 2-(2-furoyl)-4(5)-(2-furanyl)-1H-imidazole, lié à un vecteur, et leurs mélanges.

4. Composition selon la revendication 1, dans laquelle ledit vecteur est choisi dans le groupe constitué par les glucides, les protéines, les lipides, les polypeptides synthétiques, les résines naturelles et synthétiques biocompatibles, les antigènes, et leurs mélanges.

5. Composition selon la revendication 1, dans laquelle ledit agent co-stimulateur est choisi dans le groupe constitué par l'interleukine-1 et l'interféron-gamma.

6. Composition selon la revendication 1, dans laquelle ladite monokine comprend le polypeptide identifié comme étant le facteur de nécrose tumorale.

7. Composition selon la revendication 1, caractérisée en ce qu'elle est formulée afin d'administrer ledit agent par voie parentérale.

8. Composition selon la revendication 7, dans laquelle ladite voie parentérale comprend l'injection.

9. Composition selon la revendication 7, dans laquelle ladite voie parentérale comprend la cathétérisation.

10. Composition selon la revendication 1, caractérisée en ce qu'elle est formulée afin d'administrer ledit agent par voie orale.

11. Composition selon la revendication 1, dans laquelle ledit agent est préparé avec un véhicule pharmaceutiquement acceptable.

12. Composition selon la revendication 1, caractérisée en ce qu'elle est formulée afin de permettre l'administration dudit agent à la fois par voie parentérale et par voie extracorporelle audit fluide de l'organisme.

13. Composition selon la revendication 1, caractérisée en ce qu'elle est formulée afin d'être administrée à un organisme contenant une quantité d'insuline et afin de permettre le traitement dudit organisme pour abaisser son taux d'insuline actif disponible.

14. Composition pharmaceutique pour l'administration à un animal afin de favoriser la séquestration et l'élimination, de l'organisme de l'animal, d'une protéine cible ayant subi une glycosylation avancée, comprenant une quantité pharmaceutiquement efficace d'un agent capable de faire en sorte que les cellules phagocytaires dudit organisme augmentent leur activité de reconnaissance et d'élimination desdites protéines cibles, et un véhicule pharmaceutiquement acceptable, caractérisée en ce que ledit agent est choisi dans le groupe constitué par un produit terminal de glycosylation avancée lié à un vecteur, une monokine qui stimule lesdites cellules phagocytaires afin d'augmenter ladite activité de reconnaissance et d'élimination vers lesdites protéines cibles, et toute combinaison éventuelle de ce qui précède avec un agent co-stimulateur.

15. Composition pharmaceutique selon la revendication 6, dans laquelle ledit produit termina de glycosylation avancée comprend le produit de la réaction d'une macromolécule protéinée et d'un sucre.

16. Composition pharmaceutique selon la revendication 6, dans laquelle ledit produit terminal de glycosylation avancée est choisi dans le groupe constitué par le produit de la réaction de l'albumine et du glucose, le produit de la réaction de l'albumine et du glucose-6-phosphate, le chromophore fluorescent 2-(2-furoyl)-4(5)-(2-furanyl)-1H-imidazole, lié à un vecteur, et leurs mélanges.

17. Composition pharmaceutique selon la revendication 6, dans laquelle ledit vecteur est choisi dans le groupe constitué par les glucides, les protéines, les lipides, les polypeptides synthétiques, les résines naturelles et synthétiques biocompatibles, les antigènes, et leurs mélanges.

18. Composition pharmaceutique selon la revendication 6, dans laquelle ledit agent co-stimulateur est choisi dans le groupe constitué par l'interleukine-1 et l'interféron-gamma.

19. Procédé pour déterminer l'activité d'une substance que l'on considère comme susceptible de jouer le rôle d'agent pour stimuler l'organisme d'un animal pour qu'il reconnaisse et élimine de l'organisme certaines macromolécules ayant subi une glycosylation avancée, comprenant:
la préparation de plusieurs échantillons biologiques dérivés de l'organisme dudit animal contenant des cellules phagocytaires;
l'incubation d'un desdits échantillons avec l'agent prospectif étudié;
l'incubation de tout le reste, sauf un, desdits échantillons avec différents agents connus pour stimuler lesdites cellules phagocytaires contre les produits terminaux de glycosylation avancée, ledit agent étant un produit terminal de glycosylation avancée, un produit terminal de glycosylation avancée lié à un vecteur, le chromophore fluorescent 2-(2-furoyl)-4(5)-(2-furanyl)-1H-imidazole, lié à un vecteur, une monokine qui stimule lesdites cellules phagocytaires afin d'augmenter ladite activité de reconnaissance et d'élimination vers lesdites protéines cibles, toute combinaison éventuelle de ce qui précède avec un agent co-stimulateur, ou un de leurs mélanges;
la conservation du dernier desdits échantillons biologiques comme témoin;
l'introduction de chacun desdits échantillons dans une quantité aliquote individuelle d'un produit terminal de glycosylation avancée connu associé avec un marqueur convenable;
l'incubation de chacun des échantillons pendant jusqu'à environ 24 heures; et
l'observation des échantillons par la suite, pour mesurer la fixation et la décomposition dudit produit terminal de glycosylation avancée marqué, et la comparaison de l'activité des cellules incubées avec ledit agent prospectif étudié avec celle des échantillons incubés avec des agents connus, pour déterminer la présence et le degré d'activité stimulatrice dudit agent prospectif.

20. Procédé de préparation d'une composition pour le traitement d'un animal souffrant d'une pathologie causée, du moins en partie, par l'accumulation, dans l'organisme de l'animal, de macromolécules ayant subi une glycosylation avancée, comprenant l'isolement d'une quantité de cellules dudit animal, la culture d'une quantité supplémentaire desdites cellules phagocytaires à partir desdites cellules phagocytaires isolées, dans un milieu nutritif sans insuline, la récolte des cellules phagocytaires ainsi cultivées, et leur introduction dans un véhicule pharmaceutiquement acceptable capable d'être introduit dans l'organisme dudit animal.

21. Procédé pour obtenir des cellules activées capables de repousser les séquelles néfastes de l'accumulation de produits terminaux de glycosylation avancée dans l'organisme d'un animal, comprenant:
l'isolement d'une quantité de cellules phagocytaires dudit animal; et
le traitement des cellules phagocytaires ainsi isolées avec un agent capable de faire en sorte que lesdites cellules phagocytaires augmentent leur activité de reconnaissance et d'élimination des macromolécules ayant subi une glycosylation avancée.

22. Procédé de préparation d'une composition capable d'immuniser un animal contre un antigène particulier, comprenant:
la préparation d'un mélange dudit antigène et d'un agent capable de faire en sorte que les cellules phagocytaires dans l'organisme dudit animal augmentent leur activité de reconnaissance et d'élimination des macromolécules ayant subi une glycosylation avancée; et
l'obtention dudit mélange sous une forme susceptible d'être administrée à l'animal pour stimuler le développement, par le système immunitaire de l'animal, d'anticorps dudit antigène.

23. Procédé pour préparer des cellules phagocytaires activées capables d'être introduites dans l'organisme d'un animal pour immuniser l'animal contre un antigène particulier favorisant le développement in vivo d'anticorps dudit antigène, ledit procédé comprenant:
l'isolement d'une quantité de cellules phagocytaires dudit animal;
la préparation d'un mélange dudit antigène et d'un agent capable de faire en sorte que lesdites cellules phagocytaires augmentent leur activité de reconnaissance et d'élimination dès macromolécules ayant subi une glycosylation avancée; et
l'incubation des cellules phagocytaires ainsi isolées avec ledit mélange pour activer lesdites cellules phagocytaires contre celui-ci.

24. Procédé de préparation d'une composition capable d'immuniser un animal contre un antigène particulier, consistant à stimuler le développement d'anticorps dudit antigène par le système immunitaire de l'animal, ledit procédé comprenant l'étape de préparation d'un complexe couplé entre ledit antigène et un agent capable de faire en sorte que les cellules phagocytaires dans l'organisme dudit animal augmentent leur activité de reconnaissance et d'élimination des macromolécules ayant subi une glycosylation avancée.

25. Procédé pour obtenir des cellules phagocytaires activées susceptibles d'être introduites dans l'organisme d'un animal pour immuniser l'animal contre un antigène particulier, comprenant:
l'isolement d'une quantité de cellules phagocytaires dudit animal;
la préparation d'un complexe coupé entre ledit antigène et un agent capable de faire en sorte que lesdites cellules phagocytaires augmentent leur activité de reconnaissance et d'élimination des macromolécules ayant subi une glycosylation avancée; et
l'incubation des cellules phagocytaires ainsi isolées avec ledit complexe couplé pour activer lesdites cellules phagocytaires contre celui-ci.

26. Procédé selon la revendication 23 ou 25, dans lequel, avant d'être incubées avec ledit mélange ou complexe, lesdites cellules sont fixées à un substrat immobilisé et, après leur incubation avec ledit mélange ou complexe, lesdites cellules sont libérées dudit substrat.

27. Procédé pour obtenir une substance cellulaire capable d'immuniser un animal contre un antigène particulier, négativant ledit antigène, le procédé comprenant:
l'isolement d'une quantité de cellules phagocytaires dudit animal;
la préparation d'un complexe coupé entre ledit antigène et un agent capable de faire en sorte que lesdites cellules phagocytaires augmentent leur activité de reconnaissance et d'élimination des macromolécules ayant subi une glycosylation avancée;
l'incubation des cellules phagocytaires ainsi isolées avec ledit complexe couplé pour activer lesdites cellules phagocytaires contre celui-ci; et
l'isolement, dudit animal, de la substance cellulaire participant au développement de la réponse immunitaire de l'animal et l'incubation desdites cellules phagocytaires activées avec celle-ci pendant une durée et dans des conditions suffisantes pour faire en sorte que ladite substance cellulaire développe des anticorps dudit antigène.

28. Procédé selon la revendication 27, dans lequel ledit complexe coupé est fixé à un substrat immobilisé.

29. Procédé selon la revendication 27, dans lequel lesdites cellules sont fixées à un substrat immobilisé.

30. Procédé selon la revendication 24, 25 ou 27, dans lequel ledit agent est un produit terminal de glycosylation avancée, un produit terminal de glycosylation avancée lié à un vecteur, le chromophore fluorescent 2-(2-furoyl)-4(5)-(2-furanyl)-1H-imidazole lié à un vecteur, ou un de leurs mélanges.

31. Procédé selon la revendication 24, 25 ou 27, dans lequel une monokine qui stimule l'organisme pour augmenter ladite activité de reconnaissance et d'élimination vers ladite macromolécule cible est incorporée pour une co-administration avec ledit complexe coupé, lesdites cellules phagocytaires activées ou ladite substance cellulaire.

32. Procédé selon la revendication 24, 25 ou 27, dans lequel un agent co-stimulateur est incorporé pour une co-administration avec ledit complexe coupé, lesdites cellules phagocytaires activées ou ladite substance cellulaire.

33. Procédé pour obtenir le moyen d'identifier et de mesurer l'étendue de l'état pathologique chez un animal, comprenant:
l'isolement d'une quantité de cellules phagocytaires dudit animal;
la fixation auxdites cellules d'un marqueur radioactif si bien que, lorsque lesdites cellules marquées sont introduites dans l'organisme dudit animal, lesdites cellules marquées soient capables d'être détectées pour déterminer l'endroit et l'étendue de l'accumulation de produits terminaux de glycosylation avancée, ceci permettant de déterminer l'endroit et l'étendue correspondants des pathologies dont ladite accumulation est une manifestation.

34. Procédé selon la revendication 33, dans lequel, avant ladite étape d'introduction, lesdites cellules phagocytaires sont incubées avec un agent capable de faire en sorte que lesdites cellules phagocytaires augmentent leur activité de reconnaissance et d'élimination des macromolécules ayant subi une glycosylation avancée.

35. Procédé pour préparer un moyen de mesure du bilan pathologique d'un animal, comprenant:
la préparation d'un agent capable de faire en sorte que les cellules phagocytaires dudit animal augmentent leur activité de reconnaissance et d'élimination des macromolécules ayant subi une glycosylation non enzymatique avancée;
la fixation d'un marqueur approprié audit agent, si bien que, lorsque ledit agent marqué est administré audit animal, ce bilan pathologique soit indiqué par la mesure du temps nécessaire audit organisme pour reconnaître et éliminer ledit agent marqué et par la comparaison des valeurs de temps obtenues avec les valeurs de temps observées pour des animaux à l'état normal.

36. Procédé pour mesurer le bilan pathologique d'un animal, comprenant:
la préparation d'un agent capable de faire en sorte que les cellules phagocytaires dudit animal augmentent leur activité de reconnaissance et d'élimination des macromolécules ayant subi une glycosylation non enzymatique avancée;
la fixation audit agent d'un marqueur approprié;
l'isolement d'un échantillon de cellules phagocytaires dudit animal et l'incubation desdites cellules avec ledit agent marqué;
la mesure du temps nécessaire audit échantillon pour reconnaître et éliminer ledit agent marqué; et
la comparaison des valeurs de temps obtenues avec les valeurs de temps observées pour des échantillons cellulaires d'animaux à l'état normal.

37. Procédé pour mesurer l'identité et la quantité de produits terminaux de glycosylation avancée dans un échantillon cellulaire de l'organisme d'un animal, comprenant:
l'isolement d'une colonie de cellules phagocytaires dudit organisme;
l'incubation de ladite colonie avec un produit terminal de glycosylation avancée auquel est fixé un marqueur;
la mesure de la fixation et/ou de la décomposition par ladite colonie dudit produit terminal de glycosylation avancée marque;
la comparaison des résultats de ladite mesure avec les résultats dérivés de mesures identiques faites à partir de cellules sous activation normale; et
la détermination, à partir de ces résultats, du degré d'activation des cellules de ladite colonie par les produits terminaux de glycosylation avancée, et donc de la quantité et de l'identité des produits terminaux de glycosylation avancée présents dans ledit échantillon cellulaire.

38. Procédé selon les revendications 21, 22, 23 ou 27, dans lequel ledit agent est un produit terminal de glycosylation avancée, un produit terminal de glycosylation avancée lié à un vecteur, le chromophore fluorescent 2-(2-furoyl)-4(5)-(2-furanyl)-1H-imidazole lié à un vecteur, une monokine qui stimule l'organisme afin d'augmenter ladite activité de reconnaissance et d'élimination vers lesdites macromolécules cibles, toute combinaison éventuelle de ce qui précède avec un agent co-stimulateur, ou un de leurs mélanges.

39. Procédé selon la revendication 21, dans lequel lesdites cellules sont traitées de manière à être capables d'être réintroduites dans ledit organisme par voie parentérale.

40. Procédé selon la revendication 39, dans lequel ladite voie parentérale comprend l'injection.

41. Procédé selon la revendication 39, dans lequel ladite voie parentérale comprend la cathétérisation.

42. Utilisation d'une composition comprenant un mélange associé d'un anticorps à des protéines ou cellules cibles, et d'un agent capable de faire en sorte que des cellules phagocytaires chez un animal augmentent leur activité de reconnaissance et d'élimination des macromolécules ayant subi une glycosylation avancée, pour la préparation d'un mélange pour traiter une pathologie chez l'animal, avec l'affaiblissement concomitant de certaines protéines ou cellules cibles, par administration dudit mélange à l'animal pour former un complexe entre ledit mélange et lesdites protéines ou cellules cibles et stimulation des cellules phagocytaires de l'animal pour augmenter la reconnaissance spécifique et l'élimination dudit mélange, de sorte que lesdits phagocytes aient une action de reconnaissance et d'élimination dudit mélange et provoquent ainsi la destruction desdites protéines ou cellules cibles.

43. Utilisation selon la revendication 42, dans laquelle ledit agent est choisi dans le groupe constitué par un produit terminal de glycosylation avancée, un produit terminal de glycosylation avancée lié à un vecteur, une monokine qui stimule l'organisme afin d'augmenter ladite activité de reconnaissance et d'élimination vers lesdites macromolécules cibles, et toute combinaison éventuelle de ce qui précède avec un agent co-stimulateur, et leurs mélanges.

44. Utilisation selon la revendication 43, dans laquelle ledit produit terminal de glycosylation avancée comprend le produit de la réaction d'une macromolécule protéinée et d'un sucre.

45. Procédé selon la revendication 43, dans lequel ledit produit terminal de glycosylation avancée est choisi dans le groupe constitué par le produit de la réaction de l'albumine et du glucose, le produit de la réaction de l'albumine et du glucose-6-phosphate, le chromophore fluorescent 2-(2-furoyl)-4(5)-(2-furanyl)-1H-imidazole, lié à un vecteur, et leurs mélanges.

46. Utilisation selon la revendication 43, dans laquelle ledit vecteur est choisi dans le groupe constitué par les glucides, les protéines, les lipides, les polypeptides synthétiques, les résines naturelles et synthétiques biocompatibles, les antigènes, et leurs mélanges.

47. Utilisation selon la revendication 43, dans laquelle ladite monokine comprend le polypeptide identifié comme étant le facteur de nécrose tumorale.

48. Utilisation selon la revendication 43, dans laquelle ledit agent co-stimulateur est choisi dans le groupe constitué par l'interleukine-1 et l'interféron-gamma.
